# EUROPÄISCHE PATENTANMELDUNG

(11) **EP 2 219 030 A1**
(43) Veröffentlichungstag der Anmeldung: **18.08.2010**
(21) Anmeldenummer: 09002105.6
(22) Anmeldetag: 16.02.2009
(51) Int. Cl.: G01N 33/53, G01N 33/569

(54) **Vorrichtung zum serologischen Nachweis von Yersinien-Infektionen und/oder deren Folgeerkrankungen sowie Verwendung der Proteine MyfA und PsaA von Y. enterocolitica und Y. pseudotuberculosis als rekombinante Antigene**

(71) Anmelder: Mikrogen Molekularbiologische Entwicklungs-GmbH, 82061 Neuried (DE)
(72) Erfinder: Soutschek, Erwin, 83225 Berg (DE)
(74) Vertreter: Keller, Günter

(57) **Zusammenfassung**

Offenbart werden Vorrichtungen zum serologischen Nachweis einer Infektion mit *Yersinia ssp*, wobei diese Vorrichtung wenigstens ein Antigen, ausgewählt aus der Gruppe von Antigenen bestehend aus folgender Gruppe: YopD, YopH, YopM, YopE, V-AG und/oder YopN oder ein Fragment eines dieser Antigene mit mindestens acht aufeinanderfolgenden Aminosäuren und weiterhin eines von zwei Proteinen ausgewählt aus MyfA und PsaA oder Fragmente eines dieser beiden Proteine mit mindestens acht aufeinanderfolgenden Aminosäuren aufweist.

SEQUENCE LISTING

<110> Mikrogen molekularbiologische Entwicklungs - GmbH

<120> Vorrichtung zum serologischen Nachweis der humanen
Yersinien-Infektionen und/oder deren Folgeerkrankungen sowie
Verwendung der Proteine MyfA und PsaA von Y. enterocolitica und
Y. pseudotuberculosis als rekombinante Antigene

<130> MyfA,PsaA

<160> 24

<170> PatentIn version 3.5

<210> 1
<211> 306
<212> PRT
<213> Yersinia

<400> 1

Met Thr Ile Asn Ile Lys Thr Asp Ser Pro Ile Ile Thr Thr Gly Ser
1               5                   10                  15

Gln Ile Asp Ala Ile Thr Thr Glu Thr Val Gly Gln Ser Gly Glu Val
            20                  25                  30

Lys Lys Thr Glu Asp Thr Arg His Glu Ala Gln Ala Ile Lys Ser Ser
        35                  40                  45

Glu Ala Ser Leu Ser Arg Ser Gln Val Pro Glu Leu Ile Lys Pro Ser
    50                  55                  60

Gln Gly Ile Asn Val Ala Leu Leu Ser Lys Ser Gln Gly Asp Leu Asn
65                  70                  75                  80

Gly Thr Leu Ser Ile Leu Leu Leu Leu Leu Glu Leu Ala Arg Lys Ala
                85                  90                  95

Arg Glu Met Gly Leu Gln Gln Arg Asp Ile Glu Asn Lys Ala Ala Ile
            100                 105                 110

Thr Ala Gln Lys Glu Gln Val Ala Glu Met Val Ser Gly Ala Lys Leu
        115                 120                 125

Met Ile Ala Met Ala Val Val Ser Gly Ile Met Ala Ala Thr Ser Thr
    130                 135                 140

Val Ala Ser Ala Phe Ser Ile Ala Lys Glu Val Lys Ile Val Lys Gln
145                 150                 155                 160

Glu Gln Ile Leu Asn Ser Asn Ile Ala Gly Arg Asp Gln Leu Ile Asp
                165                 170                 175

Thr Lys Leu Gln Gln Met Ser Asn Thr Ser Asp Lys Ala Val Ser Arg
            180                 185                 190

Glu Asp Ile Gly Arg Ile Trp Lys Pro Glu Gln Val Ala Asp Gln Asn
        195                 200                 205

Lys Leu Ala Leu Leu Asp Lys Glu Phe Arg Met Thr Asp Ser Lys Ala
    210                 215                 220

Asn Ala Phe Asn Ala Ala Thr Gln Pro Leu Gly Gln Met Ala Asn Ser
225                 230                 235                 240

Ala Ile Gln Val His Arg Gly Tyr Ser Gln Ala Glu Val Lys Glu Lys
                245                 250                 255

Glu Val Asn Ala Ser Ile Ala Ala Asn Glu Lys Gln Lys Ala Glu Glu
            260                 265                 270

Ala Met Asn Tyr Asn Asp Asn Phe Met Lys Asp Val Leu Arg Leu Ile
        275                 280                 285

Glu Gln Tyr Val Ser Ser His Thr His Ala Met Lys Ala Ala Phe Gly
    290                 295                 300

Val Val
305

<210>  2
<211>  468
<212>  PRT
<213>  Yersinia

<400>  2

Met Asn Leu Ser Leu Ser Asp Leu His Arg Gln Val Ser Arg Leu Val
1               5                   10                  15

Gln Gln Glu Ser Gly Asp Cys Thr Gly Lys Leu Arg Gly Asn Val Ala
            20                  25                  30

Ala Asn Lys Glu Thr Thr Phe Gln Gly Leu Thr Ile Ala Ser Gly Ala
        35                  40                  45

Arg Glu Ser Glu Lys Val Phe Ala Gln Thr Val Leu Ser His Val Ala
    50                  55                  60

Asn Ile Val Leu Thr Gln Glu Asp Thr Ala Lys Leu Leu Gln Ser Thr
65                  70                  75                  80

Val Lys His Asn Leu Asn Asn Tyr Glu Leu Arg Ser Val Gly Asn Gly
                85                  90                  95

Asn Ser Val Leu Val Ser Leu Arg Ser Asp Gln Met Thr Leu Gln Asp
            100                 105                 110

Ala Lys Val Leu Leu Glu Ala Ala Leu Arg Gln Glu Ser Gly Ala Arg
        115                 120                 125

Gly His Val Ser Ser His Ser His Ser Val Leu His Ala Pro Gly Thr
    130                 135                 140

Pro Val Arg Glu Gly Leu Arg Ser His Leu Asp Pro Arg Thr Pro Pro
145                 150                 155                 160

Leu Pro Pro Arg Glu Arg Pro His Thr Ser Gly His His Gly Ala Gly
                165                 170                 175

Glu Ala Arg Ala Thr Ala Pro Ser Thr Val Ser Pro Tyr Gly Pro Glu
            180                 185                 190

Ala Arg Ala Glu Leu Ser Ser Arg Leu Thr Thr Leu Arg Asn Thr Leu
        195                 200                 205

Ala Pro Ala Thr Asn Asp Pro Arg Tyr Leu Gln Ala Cys Gly Gly Glu
    210                 215                 220

Lys Leu Asn Arg Phe Arg Asp Ile Gln Cys Cys Arg Gln Thr Ala Val
225                 230                 235                 240

Arg Ala Asp Leu Asn Ala Asn Tyr Ile Gln Val Gly Asn Thr Arg Thr
                245                 250                 255

Ile Ala Cys Gln Tyr Pro Leu Gln Ser Gln Leu Glu Ser His Phe Arg
            260                 265                 270

Met Leu Ala Glu Asn Arg Thr Pro Val Leu Ala Val Leu Ala Ser Ser
        275                 280                 285

Ser Glu Ile Ala Asn Gln Arg Phe Gly Met Pro Asp Tyr Phe Arg Gln
    290                 295                 300

Ser Gly Thr Tyr Gly Ser Ile Thr Val Glu Ser Lys Met Thr Gln Gln
305                 310                 315                 320

Val Gly Leu Gly Asp Gly Ile Met Ala Asp Met Tyr Thr Leu Thr Ile
                325                 330                 335

Arg Glu Ala Gly Gln Lys Thr Ile Ser Val Pro Val Val His Val Gly
            340                 345                 350

Asn Trp Pro Asp Gln Thr Ala Val Ser Ser Glu Val Thr Lys Ala Leu
        355                 360                 365

Ala Ser Leu Val Asp Gln Thr Ala Glu Thr Lys Arg Asn Met Tyr Glu
    370                 375                 380

Ser Lys Gly Ser Ser Ala Val Ala Asp Asp Ser Lys Leu Arg Pro Val
385                 390                 395                 400

Ile His Cys Arg Ala Gly Val Gly Arg Thr Ala Gln Leu Ile Gly Ala
                405                 410                 415

Met Cys Met Asn Asp Ser Arg Asn Ser Gln Leu Ser Val Glu Asp Met
            420                 425                 430

Val Ser Gln Met Arg Val Gln Arg Asn Gly Ile Met Val Gln Lys Asp
        435                 440                 445

Glu Gln Leu Asp Val Leu Ile Lys Leu Ala Glu Gly Gln Gly Arg Pro
    450                 455                 460

Leu Leu Asn Ser
465

<210>   3
<211>   293
<212>   PRT
<213>   Yersinia

<400>   3

Met Thr Thr Leu His Asn Ile Ser Tyr Gly Asn Thr Thr Leu Arg Asn
1               5                   10                  15

Glu His Pro Glu Thr Ala Ser Ser Gln Ile Val Asn Gln Thr Leu Gly
            20                  25                  30

Gln Phe Arg Gly Glu Ser Val Gln Ile Val Ser Gly Thr Leu Gln Ser
        35                  40                  45

Ile Ala Asp Met Ala Glu Glu Val Thr Phe Val Phe Ser Glu Arg Lys
    50                  55                  60

Glu Leu Ser Leu Asp Lys Arg Lys Leu Ser Asp Ser Gln Ala Arg Val
65                  70                  75                  80

Ser Asp Val Glu Glu Gln Val Asn Gln Tyr Leu Ser Lys Val Pro Glu
                85                  90                  95

Leu Glu Gln Lys Gln Asn Val Ser Glu Leu Leu Ser Leu Leu Ser Asn
            100                 105                 110

Ser Pro Asn Ile Ser Leu Ser Gln Leu Lys Ala Tyr Leu Glu Gly Lys
        115                 120                 125

Ser Glu Glu Pro Ser Glu Gln Phe Lys Met Leu Cys Gly Leu Arg Asp
    130                 135                 140

Ala Leu Lys Gly Arg Pro Glu Leu Ala His Leu Ser His Leu Val Glu
145                 150                 155                 160

Gln Ala Leu Val Ser Met Ala Glu Glu Gln Gly Glu Ala Ile Val Leu
                165                 170                 175

Gly Ala Arg Ile Thr Pro Glu Ala Tyr Arg Glu Ser Gln Ser Ser Val
            180                 185                 190

Asn Pro Leu Gln Pro Leu Arg Asp Thr Tyr Arg Asp Ala Val Met Gly
        195                 200                 205

Tyr Gln Gly Ile Tyr Ala Ile Trp Ser Asp Leu Gln Lys Arg Phe Pro
    210                 215                 220

Asn Gly Asp Ile Asp Ser Val Ile Leu Phe Leu Gln Lys Ala Leu Ser
225                 230                 235                 240

Ala Asp Leu Gln Ser Gln Gln Ser Gly Ser Gly Arg Glu Lys Leu Gly
                245                 250                 255

Ile Val Ile Ser Asp Leu Gln Lys Leu Lys Glu Phe Gly Ser Val Ser
            260                 265                 270

Asp Gln Val Lys Gly Phe Trp Gln Phe Phe Ser Glu Gly Lys Thr Asn
        275                 280                 285

Gly Val Arg Pro Phe
    290

<210>  4
<211>  220
<212>  PRT
<213>  Yersinia

<400>  4

Met Pro Lys Ile Ser Ser Phe Ile Ser Thr Ser Leu Pro Leu Pro Thr
1               5                   10                  15

Ser Val Ser Gly Ser Ser Ser Val Gly Glu Met Ser Gly Arg Ser Val
            20                  25                  30

Ser Gln Gln Lys Ser Glu Gln Tyr Ala Asn Asn Leu Ala Gly Arg Thr
        35                  40                  45

Glu Ser Pro Gln Gly Ser Ser Leu Ala Ser Arg Ile Thr Glu Lys Leu
    50                  55                  60

Ser Ser Met Ala Arg Ser Ala Ile Glu Phe Ile Lys Arg Met Phe Ser
65                  70                  75                  80

Glu Gly Ser His Lys Pro Val Val Thr Pro Ala Pro Thr Pro Ala Gln

                85                  90                  95

Met Pro Ser Pro Thr Ser Phe Ser Asp Ser Ile Lys Gln Leu Ala Ala
            100                 105                 110

Glu Thr Leu Pro Lys Tyr Ile Gln Gln Leu Ser Ser Leu Asp Ala Glu
        115                 120                 125

Thr Leu Gln Lys Asn His Asp Gln Phe Ala Thr Gly Ser Gly Pro Leu
    130                 135                 140

Arg Gly Ser Ile Thr Gln Cys Gln Gly Leu Met Gln Phe Cys Gly Gly
145                 150                 155                 160

Glu Leu Gln Ala Glu Ala Ser Ala Ile Leu Asn Thr Pro Val Cys Gly
                165                 170                 175

Ile Pro Phe Ser Gln Trp Gly Thr Ile Gly Gly Ala Ala Ser Ala Tyr
            180                 185                 190

Val Ala Ser Gly Val Asp Leu Thr Gln Ala Ala Asn Glu Leu Lys Gly
        195                 200                 205

Leu Ala Gln Gln Met His Gln Leu Leu Ser Leu Met
    210                 215                 220

<210> 5
<211> 505
<212> PRT
<213> Yersinia

<400> 5

Met Phe Ile Asn Pro Arg Asn Val Ser Asn Thr Phe Leu Gln Glu Pro
1               5                   10                  15

Leu Arg His Ser Ser Asp Leu Thr Glu Ile Pro Val Glu Ala Glu Asn
            20                  25                  30

Val Lys Ser Lys Thr Glu Tyr Tyr Asn Ala Trp Ser Glu Trp Glu Arg
        35                  40                  45

Asn Ala Pro Pro Gly Asn Gly Glu Gln Arg Glu Met Ala Val Ser Arg
    50                  55                  60

Leu Arg Asp Cys Leu Asp Arg Gln Ala His Glu Leu Glu Leu Asn Asn
65                  70                  75                  80

Leu Gly Leu Ser Ser Leu Pro Glu Leu Pro Pro His Leu Glu Arg Leu
                85                  90                  95

Val Ala Ser Cys Asn Ser Leu Thr Glu Leu Pro Glu Leu Pro Gln Ser
            100                 105                 110

Leu Lys Ser Leu Glu Val Tyr Glu Asn Asn Leu Lys Ala Leu Pro Asp
        115                 120                 125

Leu Pro Pro Leu Leu Val Asp Leu Arg Val Phe Asn Asn Gln Leu Glu
    130                 135                 140

Glu Leu Pro Glu Leu Gln Asn Leu Pro Phe Leu Thr Glu Ile Tyr Ala
145                 150                 155                 160

Asn Asn Asn Ser Leu Lys Thr Leu Pro Asp Leu Pro Pro Ser Leu Val
                165                 170                 175

Asp Leu Asn Val Arg Glu Asn Tyr Leu Thr Ala Leu Pro Glu Leu Pro
            180                 185                 190

Gln Ser Leu Ile Phe Leu Asp Ile Ser Asp Asn Ile Leu Ser Gly Leu
        195                 200                 205

Ser Glu Leu Pro Pro Asn Leu Ser Cys Leu Asp Ala Ser Arg Asn Gly
    210                 215                 220

Ile Arg Ser Leu Cys Asp Leu Pro Pro Ser Leu Val Tyr Leu Asp Val
225                 230                 235                 240

Arg Asp Asn Gln Leu Ile Glu Leu Pro Ala Leu Pro Ser Gly Leu Glu
                245                 250                 255

Arg Leu Ile Ala Ser Phe Asn His Leu Ala Glu Leu Pro Glu Leu Pro
            260                 265                 270

Pro Asn Leu Tyr Tyr Leu Asp Ala Ser Arg Asn Glu Ile Ser Ser Leu
        275                 280                 285

Cys Asp Leu Pro Pro Ser Leu Val Asp Leu Asn Val Arg Lys Asn Gln
    290                 295                 300

Leu Ile Glu Leu Pro Ala Leu Pro Pro Asp Leu Glu Arg Leu Ile Ala
305                 310                 315                 320

Ser Phe Asn His Leu Ala Glu Leu Pro Glu Leu Pro Pro Asn Leu Ser
                325                 330                 335

Tyr Leu Asp Ala Ser Arg Asn Glu Ile Ser Ser Leu Cys Asp Leu Pro
            340                 345                 350

Pro Ser Leu Val Asp Leu Asn Val Arg Lys Asn Gln Leu Ile Glu Leu
        355                 360                 365

Pro Ala Leu Pro Pro Asp Leu Glu Arg Leu Ile Ala Ser Phe Asn His
    370                 375                 380

Leu Ala Glu Leu Pro Glu Leu Pro Pro Asn Leu Ser Tyr Leu Asp Ala
385                 390                 395                 400

Ser Arg Asn Glu Ile Ser Ser Leu Cys Asp Leu Pro Pro Ser Leu Val
                405                 410                 415

Glu Leu Asp Val Arg Asp Asn Gln Leu Ile Glu Leu Pro Ala Leu Pro
            420                 425                 430

Pro His Leu Glu Arg Leu Ile Ala Ser Leu Asn His Leu Ala Glu Val
        435                 440                 445

Pro Glu Leu Pro Gln Asn Leu Lys Gln Leu His Val Glu His Asn Ala
    450                 455                 460

Leu Arg Glu Phe Pro Asp Ile Pro Glu Ser Val Glu Asp Leu Arg Met
465                 470                 475                 480

Asp Ser Glu Arg Val Ile Asp Pro Tyr Glu Phe Ala His Glu Thr Ile
                485                 490                 495

Asp Lys Leu Glu Asp Asp Val Phe Glu
            500                 505

<210>  6
<211>  333
<212>  PRT
<213>  Yersinia

<400>  6

Met Ile Arg Ala Tyr Glu Gln Asn Pro Gln His Phe Ile Glu Asp Leu
1               5                   10                  15

Glu Lys Val Arg Val Glu Gln Leu Thr Gly His Gly Ser Ser Val Leu
            20                  25                  30

Glu Glu Leu Val Gln Leu Val Lys Asp Lys Lys Ile Asp Ile Ser Ile
        35                  40                  45

Lys Tyr Asp Pro Lys Lys Asp Ser Glu Val Phe Ala Glu Arg Val Ile
    50                  55                  60

Thr Asp Asp Ile Glu Leu Leu Lys Lys Ile Leu Ala Tyr Phe Leu Pro
65                  70                  75                  80

Glu Asp Ala Ile Leu Lys Gly Gly His Tyr Asp Asn Gln Leu Gln Asn
                85                  90                  95

Gly Ile Lys Arg Val Lys Glu Phe Leu Glu Ser Ser Pro Asn Thr Gln
            100                 105                 110

Trp Glu Leu Arg Ala Phe Met Ala Val Met His Phe Ser Leu Thr Ala
        115                 120                 125

Asp Arg Ile Asp Asp Asp Ile Leu Lys Val Ile Val Asp Ser Met Asn
    130                 135                 140

His His Gly Asp Ala Arg Ser Lys Leu Arg Glu Glu Leu Ala Glu Leu
145                 150                 155                 160

Thr Ala Glu Leu Lys Ile Tyr Ser Val Ile Gln Ala Glu Ile Asn Lys
                165                 170                 175

His Leu Ser Ser Ser Gly Thr Ile Asn Ile His Glu Lys Ser Ile Asn
            180                 185                 190

Leu Met Asp Lys Asn Leu Tyr Gly Tyr Thr Asp Glu Glu Ile Phe Lys
        195                 200                 205

Ala Ser Ala Glu Tyr Lys Ile Leu Lys Lys Met Pro Gln Thr Thr Ile
    210                 215                 220

Lys Asp Asp Glu Leu His Glu Val Gly Val Ile Ala Gly Ala Glu Lys
225                 230                 235                 240

Gln Ile Val Ser Ile Lys Asn Phe Leu Glu Ser Glu Asn Lys Arg Thr
                245                 250                 255

Gly Ala Leu Gly Asn Leu Lys Asp Ser Tyr Ser Tyr Asn Lys Asp Asn
            260                 265                 270

Asn Glu Leu Ser His Phe Ala Thr Ala Cys Ser Asp Lys Ser Arg Pro
        275                 280                 285

Leu Asn Asp Leu Val Ser Gln Lys Thr Thr Gln Leu Ser Asp Ile Thr
    290                 295                 300

Ser Arg Phe Asn Ser Ala Ile Glu Ala Leu Asn Arg Phe Ile Gln Lys
305                 310                 315                 320

Tyr Asp Ser Val Met Gln Arg Leu Leu Asp Asp Thr Arg
                325                 330

<210>  7
<211>  131
<212>  PRT
<213>  Yersinia

<400>  7

Met Glu Pro Thr Val Ile Asn Ser Lys Asp Ile Ser Ala Thr Lys Thr
1               5                   10                  15

Val Lys Glu Gly Gly Ser Phe Ser Val Glu Phe Lys Ala Thr Glu Asn
            20                  25                  30

Glu Ile Val Ser Gly Lys Leu Asp Ala Asp Thr Pro Ala Phe His Leu
        35                  40                  45

Val Met Ser Asp Ser Gly Glu His Lys Gly Trp Asn Val Arg Pro Thr
    50                  55                  60

Gly Ala Ser Glu Gly Gly Gln Met Val Ser Ala Asp Gly Thr Arg Val
65                  70                  75                  80

Asp Leu His Thr Asn Glu Leu Ser Trp Asp Asn Asp His Trp Trp Ile
                85                  90                  95

Asp Asp Gly Ser Glu Arg Val Glu Ala Thr Phe Phe Leu Ala Ala Gly
            100                 105                 110

Asp Glu Val Lys Ala Gly Glu Tyr Gln Phe Thr Gly Arg Val Glu Glu
        115                 120                 125

Tyr Val Glu
    130

<210>  8
<211>  133
<212>  PRT
<213>  Yersinia

<400>  8

Met Ser Thr Val Ile Asn Ser Lys Asp Val Ser Gly Glu Val Thr Val
1               5                   10                  15

Lys Gln Gly Asn Thr Phe His Val Asp Phe Ala Pro Asn Thr Gly Glu
            20                  25                  30

Ile Phe Ala Gly Lys Gln Pro Gly Asp Val Thr Met Phe Thr Leu Thr
        35                  40                  45

Met Gly Asp Thr Ala Pro His Gly Gly Trp Arg Leu Ile Pro Thr Gly
    50                  55                  60

Asp Ser Lys Gly Gly Tyr Met Ile Ser Ala Asp Gly Asp Tyr Val Gly
65                  70                  75                  80

Leu Tyr Ser Tyr Met Met Ser Trp Val Gly Ile Asp Asn Asn Trp Tyr
                85                  90                  95

Ile Asn Asp Asp Ser Pro Lys Asp Ile Lys Asp His Leu Tyr Val Lys
            100                 105                 110

Ala Gly Thr Val Leu Lys Pro Thr Thr Tyr Lys Phe Thr Gly Arg Val

115                 120                 125

Glu Glu Tyr Val Phe
    130

<210>  9
<211>  396
<212>  DNA
<213>  Yersinia

<400>  9
atggaaccga ctgttattaa tagtaaagac atctctgcaa caaaaactgt taaagaggga      60

ggttcgttct cagttgaatt caaggccact gaaaacgaga ttgtgtcagg caaattggat     120

gcagatacac ctgccttcca tctggtaatg tcggactcag gggaacataa aggttggaat      180

gttcggccta ccggtgcatc tgagggagga cagatggttt ctgcagatgg taccagagtt     240

gacttacata caaatgagct atcgtgggat aacgaccact ggtggataga tgacggttct     300

gagcgtgtgg aagcgacttt ctttcttgct gctggcgacg aggttaaagc aggtgaatat     360

cagttcactg ggcgtgttga ggaatatgtc gagtaa                               396

<210>  10
<211>  402
<212>  DNA
<213>  Yersinia

<400>  10
atgtctactg tcattaactc caaggatgtt tctggtgagg tgactgtcaa gcagggaaac      60

acattccacg tcgattttgc gcctaacaca ggagagattt ttgcgggtaa acagccgggt     120

gatgtcacta tgtttacgct aactatgggt gatactgcac cacacggtgg ttggcgtttg     180

attccaacag gggactcaaa aggtggatat atgatcagcg ccgatggtga ctatgttggt     240

ttatacagtt atatgatgtc atgggtaggt atagataata actggtatat aaatgatgac     300

tctcctaaag atataaaaga tcatctgtac gttaaggcag ggactgtcct taaaccaacg     360

acttataaat tcacggggcg tgttgaagag tatgtatttt aa                        402

<210>  11
<211>  159
<212>  PRT
<213>  Yersinia

<400>  11

Met Asn Met Lys Lys Phe Val Lys Lys Pro Leu Ala Ile Ala Val Leu
1               5                   10                  15

Met Leu Ala Ser Gly Gly Met Val Asn Met Val His Ala Glu Pro Thr
            20                  25                  30

Val Ile Asn Ser Lys Asp Ile Ser Ala Thr Lys Thr Val Lys Glu Gly
        35                  40                  45

Gly Ser Phe Ser Val Glu Phe Lys Ala Thr Glu Asn Glu Ile Val Ser
    50                  55                  60

Gly Lys Leu Asp Ala Asp Thr Pro Ala Phe His Leu Val Met Ser Asp
65                  70                  75                  80

Ser Gly Glu His Lys Gly Trp Asn Val Arg Pro Thr Gly Ala Ser Glu
                85                  90                  95

Gly Gly Gln Met Val Ser Ala Asp Gly Thr Arg Val Asp Leu His Thr
            100                 105                 110

Asn Glu Leu Ser Trp Asp Asn Asp His Trp Trp Ile Asp Asp Gly Ser
        115                 120                 125

Glu Arg Val Glu Ala Thr Phe Phe Leu Ala Ala Gly Asp Glu Val Lys
    130                 135                 140

Ala Gly Glu Tyr Gln Phe Thr Gly Arg Val Glu Glu Tyr Val Glu
145                 150                 155

<210>  12
<211>  158
<212>  PRT
<213>  Yersinia

<400>  12

Met Lys Met Lys Cys Phe Ala Lys Asn Ala Leu Ala Val Thr Thr Leu
1               5                   10                  15

Met Ile Ala Ala Cys Gly Met Ala Asn Ala Ser Thr Val Ile Asn Ser
            20                  25                  30

Lys Asp Val Ser Gly Glu Val Thr Val Lys Gln Gly Asn Thr Phe His
        35                  40                  45

Val Asp Phe Ala Pro Asn Thr Gly Glu Ile Phe Ala Gly Lys Gln Pro
    50                  55                  60

Gly Asp Val Thr Met Phe Thr Leu Thr Met Gly Asp Thr Ala Pro His
65                  70                  75                  80

Gly Gly Trp Arg Leu Ile Pro Thr Gly Asp Ser Lys Gly Gly Tyr Met
                85                  90                  95

Ile Ser Ala Asp Gly Asp Tyr Val Gly Leu Tyr Ser Tyr Met Met Ser
            100                 105                 110

Trp Val Gly Ile Asp Asn Asn Trp Tyr Ile Asn Asp Asp Ser Pro Lys
        115                 120                 125

Asp Ile Lys Asp His Leu Tyr Val Lys Ala Gly Thr Val Leu Lys Pro

130                 135                 140

Thr Thr Tyr Lys Phe Thr Gly Arg Val Glu Glu Tyr Val Phe
145                 150                 155

<210>  13
<211>  30
<212>  DNA
<213>  Artificial Sequence

<220>
<223>  primer

<400>  13
gtaattccat atgaatatga aaaaatttgt                                        30

<210>  14
<211>  27
<212>  DNA
<213>  Artificial Sequence

<220>
<223>  primer

<400>  14
gtaatcccat atggcaacaa aaactgt                                           27

<210>  15
<211>  24
<212>  DNA
<213>  Artificial Sequence

<220>
<223>  primer

<400>  15
ttactcgagt tcacctgctt taac                                              24

<210>  16
<211>  28
<212>  DNA
<213>  Artificial Sequence

<220>
<223>  primer

<400>  16
atctactcga gctcgacata ttcctcaa                                          28

<210>  17
<211>  38
<212>  PRT
<213>  Artificial Sequence

<220>
<223>  primer

<400>  17

Cys Ala Cys Ala Thr Ala Thr Gly Gly Ala Ala Cys Cys Gly Ala Cys
1               5                   10                  15

Thr Gly Thr Thr Ala Thr Thr Ala Ala Thr Ala Gly Thr Ala Ala Ala
            20                  25                  30

Gly Ala Cys Ala Thr Cys
        35

<210>  18
<211>  32
<212>  DNA
<213>  Artificial Sequence

<220>
<223>  primer

<400>  18
atggatcctt actcgacata ttcctcaaca cg                                    32

<210>  19
<211>  33
<212>  DNA
<213>  Artificial Sequence

<220>
<223>  primer

<400>  19
gccatatgtc tactgtcatt aactccaagg atg                                   33

<210>  20
<211>  34
<212>  DNA
<213>  Artificial Sequence

<220>
<223>  primer

<400>  20
caggatcctt aaaatacata ctcttcaaca cgcc                                  34

<210>  21
<211>  396
<212>  DNA
<213>  Yersinia

<400>  21
atggaaccga ctgttattaa tagtaaagac atctctgcaa caaaaactgt taaagaggga      60

ggttcgttct cagttgaatt caaggccact gaaaacgaga ttgtgtcagg caaattggat     120

gcagatacac ctgccttcca tctggtaatg tcggactcag gggaacataa aggttggaat     180

gttcggccta ccggtgcatc tgagggagga cagatggttt ctgcagatgg taccagagtt     240

gacttacata caaatgagct atcgtgggat aacgaccact ggtggataga tgacggttct     300

gagcgtgtgg aagcgacttt ctttcttgct gctggcgacg aggttaaagc aggtgaatat     360

cagttcactg ggcgtgttga ggaatatgtc gagtaa                               396

<210>  22
<211>  402

<212>  DNA
<213>  Yersinia

<400>  22
atgtctactg tcattaactc caaggatgtt tctggtgagg tgactgtcaa gcagggaaac      60

acattccacg tcgattttgc gcctaacaca ggagagattt ttgcgggtaa acagccgggt     120

gatgtcacta tgtttacgct aactatgggt gatactgcac cacacggtgg ttggcgtttg     180

attccaacag gggactcaaa aggtggatat atgatcagcg ccgatggtga ctatgttggt     240

ttatacagtt atatgatgtc atgggtaggt atagataata actggtatat aaatgatgac     300

tctcctaaag atataaaaga tcatctgtac gttaaggcag ggactgtcct taaaccaacg     360

acttataaat tcacggggcg tgttgaagag tatgtatttt aa                        402

<210>  23
<211>  131
<212>  PRT
<213>  Yersinia

<400>  23

Met Glu Pro Thr Val Ile Asn Ser Lys Asp Ile Ser Ala Thr Lys Thr
1               5                   10                  15

Val Lys Glu Gly Gly Ser Phe Ser Val Glu Phe Lys Ala Thr Glu Asn
            20                  25                  30

Glu Ile Val Ser Gly Lys Leu Asp Ala Asp Thr Pro Ala Phe His Leu
        35                  40                  45

Val Met Ser Asp Ser Gly Glu His Lys Gly Trp Asn Val Arg Pro Thr
    50                  55                  60

Gly Ala Ser Glu Gly Gly Gln Met Val Ser Ala Asp Gly Thr Arg Val
65                  70                  75                  80

Asp Leu His Thr Asn Glu Leu Ser Trp Asp Asn Asp His Trp Trp Ile
                85                  90                  95

Asp Asp Gly Ser Glu Arg Val Glu Ala Thr Phe Phe Leu Ala Ala Gly
            100                 105                 110

Asp Glu Val Lys Ala Gly Glu Tyr Gln Phe Thr Gly Arg Val Glu Glu
        115                 120                 125

Tyr Val Glu
    130

<210>  24
<211>  133
<212>  PRT
<213>  Yersinia

<400>  24

Met Ser Thr Val Ile Asn Ser Lys Asp Val Ser Gly Glu Val Thr Val
1               5                   10                  15

Lys Gln Gly Asn Thr Phe His Val Asp Phe Ala Pro Asn Thr Gly Glu
            20                  25                  30

Ile Phe Ala Gly Lys Gln Pro Gly Asp Val Thr Met Phe Thr Leu Thr
        35                  40                  45

Met Gly Asp Thr Ala Pro His Gly Gly Trp Arg Leu Ile Pro Thr Gly
    50                  55                  60

Asp Ser Lys Gly Gly Tyr Met Ile Ser Ala Asp Gly Asp Tyr Val Gly
65                  70                  75                  80

Leu Tyr Ser Tyr Met Met Ser Trp Val Gly Ile Asp Asn Asn Trp Tyr
                85                  90                  95

Ile Asn Asp Asp Ser Pro Lys Asp Ile Lys Asp His Leu Tyr Val Lys
            100                 105                 110

Ala Gly Thr Val Leu Lys Pro Thr Thr Tyr Lys Phe Thr Gly Arg Val
        115                 120                 125

Glu Glu Tyr Val Phe
    130

## Beschreibung

Das Genus *Yersinia* umfasst drei humanpathogene *Yersinia* spp., *Y. pestis, Y. pseudotuberculosis* und *Y. enterocolitica* und acht weitere Species wie *Y. aldovae, Y. bercovieri, Y. frederiksenii, Y. intermedia, Y. kristensenii, Y. moolaretti, Y. rohdei* und *Y. ruckeri,* die eher als opportunistische Krankheitserreger bei Wunden und Sepsis beim Menschen eine Rolle spielen. *Y. pestis* ist der Erreger der Pest und kann durch Flöhe von dem natürlichen Nagetier-Reservoir auf den Menschen übertragen werden. *Y. enterocolitica* und *Y. pseudotuberculosis* treten weltweit in den gemäßigten bis subtropischen Klimazonen bei Wild- und Nutztieren auf. In Deutschland ist die durch *Y. enterocolitica* und *Y. pseudotuberculosis* hervorgerufene Darmerkrankung, die sog. Yersiniose, nach der Salmonellose (*Salmonella enterica*) und Campylobacter-Enteritis (*Campylobacter jejuni*) die dritthäufigste bakterielle Enteritiserkrankung, die dem Robert Koch-Institut (RKI) Berlin jährlich gemeldet wird. Im Jahr 2007 wurden in Deutschland 4.987 und im Jahr 2006 5.162 durch Yersinien bedingte Erkrankungen gemäß RKI-Falldefinition an das Robert Koch - Institut übermittelt. Die höchste altersspezifische Inzidenz wurde bei Kleinkindern im Alter von 1 bis 4 Jahren beobachtet (RKI). Die tatsächliche Häufigkeit dieser Erkrankungen ist allerdings nicht genau bekannt - viele Erkrankte suchen bei subklinischen bzw. bei leichten und kurzen Krankheitsverläufen keinen Arzt auf, viele Erkrankungen werden ätiologisch nicht geklärt und nicht alle diagnostizierten Erkrankungsfälle werden gemeldet. Schätzungsweise bis zu 43% der Blutspender in Deutschland und 31% der Blutspender in Finnland haben spezifische Antikörper gegen Yersinien (getestet mit Hilfe von EnzymImmunoassays bzw. Immunoblots). Dieses Ergebnis lässt eine unerwartet hohe Prävalenz von durchgemachten Yersiniosen in Industrieländern mit hohen Hygienestandards vermuten.

Die enteropathogenen *Y. enterocolitica-* und *Y. pseudotuberculosis* -Erreger kommen bei Schweinen, Schafen, Rindern und Geflügel vor und gelangen hauptsächlich durch den Schlachtprozess in Lebensmittel (z.B. rohes Schweinefleisch) und Trinkwasser. Darüber hinaus sind Übertragungen auf Haustiere beschrieben. Auch konnte festgestellt werden, dass bis zu 100% der Wildschweine *Yersinia enterocolitica* im Nasenrachenraum tragen. Yersinien besitzen die Fähigkeit, sich bei niedrigen Temperaturen (> 4°C) zu vermehren. In den Industrieländern stellt die Vermehrung in gekühlten Lebensmitteln die epidemiologisch wichtigste Verbreitungsform von *Y. enterocolitica* und *Y. pseudotuberculosis* dar.

Die genaue Infektionsdosis ist nicht bekannt (vermutlich >10000 Bakterien). Die Inkubationszeit beträgt zwei bis fünf Tage nach der Ansteckung. Enteropathogene *Y. enterocolitica* und *Y. pseudotuberculosis* -Stämme siedeln sich meist im darmassoziierten lymphatischen Gewebe des terminalen Ileums, in den so genannten Peyer'schen Plaques (zusammenhängende Ansammlung von Lymphfollikeln), an. Nach Ansiedlung überwinden die Yersinien über sog. M-Zellen in der Follikel-assoziierten Epithelzellschicht der Peyer'schen Plaques die gastrointestinale Barriere des Organismus und breiten sich über die abfließenden Lymphgefäße in den mesenterialen Lymphknoten aus. Durch *Y. enterocolitica* werden nachfolgend weitere Darmbereiche befallen und geschädigt, was die typischen Gewebsverletzungen des Dünn- und Dickdarms bei Yersiniose-Patienten erklärt. *Y. pseudotuberculosis* zeigt einen ähnlichen Infektionsverlauf wie *Y. enterocolitica,* wobei aber häufiger mesenteriale Lymphknoten, Leber und Milz betroffen sind und Enteritissymptomatik und die Ausscheidung über den Darm viel geringer sind als bei *Y. enterocolitica.*

Die enteritische Form der Yersinien-Infektionen, auch als Yersiniose bezeichnet, ist in unkomplizierten Fällen auf den Magen-Darm-Trakt begrenzt und durch mehrtägigen wässrigen Durchfall gekennzeichnet (sog. akute Yersinien-Gastroenteritis). Jedoch können Yersiniosen in Abhängigkeit vom Alter, dem Immunstatus, dem Histokompatibilitätstyp (HLA-B27) und dem Geschlecht der Patienten unterschiedliche Verlaufsformen und Symptome aufzeigen, wie z.B. Fieber, Übelkeit, kolikartige Unterbauchschmerzen (Pseudoappendizitis), Muskel- und Kopfschmerzen und Entzündungen im Rachenbereich (Pharyngitis), in den mesenteriale Lymphknoten (akute mesenteriale Lymphadenitis) und im Ileum und Kolon (Yersinia-Iletitis und -Colitis). In unkomplizierten Fällen klingt die Erkrankung nach wenigen Tagen bis spätestens nach zwei Wochen ab.

Bei Patienten mit Grunderkrankungen wie z. B. *Diabetes mellitus,* Leberzirrhose, Eisenspeicherkrankheiten (Dialyse-Patienten, Thalassämie) oder einer Immunsuppression kann es zu extraintestinalen Infektionen kommen (z. B. septische Formen). Nicht selten kommt es nach einer Bluttransfusion mit Yersinia-kontaminierten Blutkonserven zum septischen Schock mit hoher Letalität (ca.75%).

Neben den intestinalen und extraintestinalen Formen der Yersiniose, bei denen der Erreger meist isoliert werden kann, treten in bis zu 30% der Fälle noch Begleit- oder Folgeerkrankungen auf, die meist einen aseptischen Verlauf haben. Die häufigste Folgeerkrankung ist die HLA-B27-assoziierte Reaktive Arthritis [kurz: ReA] (etwa 60-80% der ReA -Fälle sind HLA-B27 positiv). Die ReA beginnt ca. 1-4 Wochen nach dem Auftreten der intestinalen Symptome (oft aber ohne vorangegangener Darmsymptomatik). Dabei sind am häufigsten die Gelenke der unteren Extremitäten betroffen. In den meisten Fällen klingt die Symptomatik nach ca. 3-12 Monaten ab, der Verlauf kann sich aber auch chronisch oder rezidivierend entwickeln. Weitere häufig auftretende Komplikationen nach einer Yersiniose sind Entzündungen der Schilddrüse (Thyroiditis), des Herzmuskels (Myokarditis) und der Nieren (Glomerulonephritis), rötliche Hauterscheinungen (Erythem) in den unteren Extremitäten (*Erythema nodosum*) und akute oder chronische Vergrößerungen der Lymphknoten (Lymphadenopathien) oder der Milz (Splenomegalien).

Bei Yersiniosen ohne weitere immunologische Komplikationen, wie z. B. reaktive Arthritis, kommt es meist nach kurzer Zeit zum Abklingen der Symptome. Damit ist eine Behandlung einer Yersinien-Infektion mit Antibiotika in den meisten Fällen unnötig. Bei septischen Verläufen oder lang anhaltenden Symptomen ist eine differenzierte Behandlung mit Antibiotika notwendig. Wegen der häufig vorkommenden Betalaktamasen bei Yersinien werden Infektionen mit *Y. enterocolitica* oder *Y. pseudotuberculosis* erregerspezifisch mit Gyrasehemmern, Tetracyclin, Trimethoprim/Sulfmethoxazol u.a. behandelt.

Die Unterscheidung verwandter Yersinien-Arten (z. B. *Y. enterocolitica* und *Y. pseudotuberculosis*) erfolgt biochemisch. *Y. pseudotuberculosis* bildet dabei eine biochemisch einheitliche Gruppe, die sich in acht Serogruppen aufteilt, von denen in Europa die Serovare I, II und III von humanmedizinischer Bedeutung sind (Bottone, [1997] Clin. Microbiol. Rev., 10, 257-276). Dagegen ist *Y. enterocolitica* eine sowohl biochemisch wie auch serologisch äußerst heterogene Art, bei der pathogene und apathogene geographisch getrennte Untergruppen vorkommen. *Y. enterocolitica* wird in sechs Biovare(BV)/Biotypen mit unterschiedlichen biochemischen Leistungen (1A, 1B, 2, 3, 4. 5) und ca. 60 Serovare (anhand unterschiedlicher O- und H-Antigene) (Wauters et al., [1987] Contrib. Microbiol. Immunol., 9, 14-21) unterteilt. Während Biovar 1A Yersinien hauptsächlich in der Umwelt vorkommen, gehören die bekannten humanpathogenen *Y. enterocolitica* -Stämme zu den BV 1B (Serotypen O:8, O:4, O:13, O:18, O:20, O:21, hauptsächliches Vorkommen in den USA), BV 2 (Europa [O:9], USA, Japan [O:5, 27]), BV 3 (O:9 und O:5, 27) und BV 4 (Europa und USA [O:3]). (Bucher et al., [2008] Foodborne Pathog. Dis., 5, 273-280). Ca. 80-90% der gemeldeten Yersinien-Gastroenteritiden in Deutschland werden durch *Y. enterocolitica -* Serotyp O:3 verursacht (RKI).

Die Virulenz der enteropathogenen Yersinien *(Y. enterocolitica* Biovare 1 B, 2, 3, 4 und 5, *Y. pseudotuberculosis)* und des Pesterregers *Y. pestis* wird durch ein hoch konserviertes 65- - 70-kp großes Virulenzplasmid pYV (in *Y. pestis* pCDI) hervorgerufen. Dessen Virulenzfaktoren ermöglichen pathogenen Yersinien im lymphoidalen Gewebe des Wirtes zu überleben. Der *Y. enterocolitica* -Biovar 1A trägt kein pYV-Plasmid und wird daher als apathogen betrachtet.

Das pYV-Plasmid kodiert für das Membranprotein YadA (*Yersinia* Adhäsin) sowie eine Reihe von sog. *Yersinia* outer proteins (Yops) und den zugehörigen Sekretionsapparat (T3SS), der als Ysc (Yop secretion) bezeichnet wird. Als Substrat des T3SS werden die Yops als Anti-Wirts-Effektoren in eukaryontische Zellen (z. B. in Makrophagen) injiziert. Der Proteinkomplex des T3SS besteht aus 27 Proteinen mit bekannten oder unbekannten Funktionen, wie z. B. einer regulatorischen Funktion (YopQ, YopN), Translokationsfunktion (YopB, YopD) und Effektorfunktion (YopH, YopE, YopT, YopP, YopO). Das Adhäsin YadA wird ebenfalls auf dem Virulenzplasmid kodiert. Es vermittelt die Adhäsion der Erreger an Wirtszellen und ermöglicht die Yops in die Zielzellen zu injizieren. Das sogenannte V-Antigen (LcrV) ist ebenfalls auf dem pYV-Plasmid kodiert. Dieses Antigen bildet die Spitze des Ysc-Injektisoms, reguliert die Yop-Sekretion und Modulation des Immunsystems des Wirts.

Die Virulenz der Yersinien wird abgesehen von den plasmidkodierten Virulenzfaktoren auch durch chromosomal kodierte Faktoren beeinflusst. Die Adhäsion der Yersinien an Epithelzellen des Darms erfordert unter anderem das Vorhandensein des Invasins Inv und des Adhäsins Ail (sog. "attachment invasion locus" der *Y. enterocolitica*), welche von allen enteropathogenen Yersinien exprimiert werden können.

Weitere bekannte chromosomal kodierte Pathogenitätsfaktoren sind z. B. das sezernierte und hitzestabile Enterotoxin Yst, das sog "Mucoid *Yersinia* factor" MyfA von *Y. enterocolitica,* bzw. das homologe pH 6-Antigen PsaA von *Y. pseudotuberculosis* und *Y. pestis,* das auf der sogenannten Hochpathogenitätsinsel (HPI) kodierte Yersiniabactin-Siderophorsystem (FyuA-Rezeptor mit Irp1-9), der Ysa Type-III-Sekretionsapparat, das für alle gram-negativen Bakterien charakteristische Lipopolysaccharid LPS, das Enzym Urease und der auf Insekten wirkende Toxinkomplex TC (*tcbA, tcaC, tccC*).

Das MyF-Fibrillensystem besteht aus den drei Untereinheiten MyfA, MyfB und MyfC. Die Fibrille wird aus MyfA-Untereinheiten aufgebaut, während die MyfB- und MyfC den Transport- und Aufbauapparat bilden. Das ca. 17-kD-große MyfA-Oberflächenantigen wird *in vitro* während der frühen stationären Wachstumsphase exprimiert, und die volle Expression erfolgt nur bei 37°C in saurem Milieu (pH 6). Das *myfA*-Gen ist in humanpathogenen *Y. enterocolitca* -Stämmen (z. B. O:3, O:4, O:8; O:9) und in ca. 16% der nichthumanpathogenen *Y. enterocolitica* BV 1A -Stämmen vorhanden und zeigt einen signifikanten Zusammenhang mit der Virulenz. Leiva und Mitarbeiter zeigten in Koagglutination-Experimenten mit Seren aus Kaninchen, die mit lebenden *Y. enterocolitica-* oder *Y. pseudotuberculosis-Stämmen* intravenös immunisiert wurden, dass die erhaltenen Antiseren gegen MyfA (*Y. enterocolitica*) bzw. PsaA (*Y. pseudotuberculosis*) eine serologische Differenzierung zwischen MyfA und PsaA erlauben (Leiva et al., [1995] Contrib. Microbiol. Immunol., 13, 158-164). Diese Immunisierungsexperimente zeigen auch, dass MyfA bzw. PsaA wahrscheinlich unter Infektionsbedingungen produziert werden und die Produktion der Serumantikörper in Kaninchen induzieren. Eine diagnostische Anwendung beim Menschen wird jedoch nicht erwähnt. Es kann auch nicht ohne weiteres erwartet werden, dass tierexperimentelle Daten auf die diagnostische Situation am Menschen übertragbar sind, weil bekanntermaßen die Produktion von Antikörpern in Tieren sich von der im Menschen unterscheiden kann.

Der Psa-Antigenkomplex (pH6-Antigen) von enteropathogenen *Y. pseudotuberculosis* wurde ursprünglich im Pesterreger *Y. pestis* als fimbrilläre Struktur mit einem Durchmesser von 3-5 nm charakterisiert. Die PsaA-Untereinheiten werden auf der Bakterienoberfläche durch den Translokations- und Aufbauapparat, der aus PsaB und PsaC besteht, angeordnet. Die ca. 17-kD-große Antigendomäne PsaA zeigt ca. 44-47%-ige Aminosäuresequenzhomologie mit den *Y.* enterocolitica-homologen MyfA. Wie beim MyfA wird die Produktion von PsaA in Yersinien durch die Temperatur (37°C) und den leicht sauren pH (pH 6) induziert. Die PsaA-Deletionmutantenstämme von *Y. pestis* zeigen eine signifikant reduzierte Virulenz *in vitro* und *in vivo.* Jedoch sind die genauen Funktionen von Myf und Psa in der Yersinieninfektion noch weitergehend unbekannt.

Die klassische Diagnose der akuten Yersiniose-Erkrankungen stützt sich primär auf den Erregernachweis im Stuhl, z. B. mittels Kälteanreicherung, selektivem Nährboden (z. B. sog. cefsulodin-Irgasan™-novobiocin -Agarnährboden [CIN-Agar]), biochemischen Eigenschaften (z. B. sog. API E20 -Test; bioMerieux, Paris, Frankreich) und den Nachweis von Erreger-spezifischer Nukleinsäuren (DNA) mittels Polymerase-Ketten-Reaktion (PCR, engl. "polymerase chain reaction"). Der Keimnachweis im Stuhl kann über eine Zeitdauer von 2 bis 12 Wochen möglich sein. Nach Abklingen der Durchfallsymptomatik sind Yersinien im Stuhl typischerweise nicht mehr nachzuweisen.

Die Serologie, d. h. der Nachweis der individuellen Serumantikörperantwort gegen Yersinien-spezifische O- und H-Antigene (sog. Widal- und passive Hämagglutinations-Tests), virulenzassoziierte Proteine (z. B. mittels Enzyme-linked Immunosorbent Assay [ELISA] - und Immunoblot -Teste) oder Bakterienultrasonikat (mittels Komplementbindungsreaktion [KBR]), ist nach geltenden Standards zur ergänzenden Diagnostik der akuten Infektion geeignet.

Jedoch ist die Serodiagnostik für die Aufklärung von Folgeerkrankungen, wie z. B. der reaktiven Arthritis, essentiell, weil der direkte Erregernachweis meistens nach Abklingen der akuten Infektion nicht möglich ist. Dir vorliegende Erfindung betrifft Vorrichtungen zur Diagnostik mit deren Hilfe zwischen einer Infektion mit *Yersinia enterocolitica* von einer Infektion mit *Yersinia pseudotuberculosis* unterschieden werden kann.

Bei einer akuten Yersinien-Infektion sind typischerweise die Yersinia-spezifische Immunoglobulin (Ig) -Klassen IgM-, IgA- und meist auch IgG nachweisbar. Im Verlauf der Infektion schwächt sich die spezifische IgM- und IgA -Antwort innerhalb von 3-6 Monaten ab (Persistenz von IgM: ca. 1-3 Monate und IgA: ca. 2-4 Monate), während Yersinien-spezifische IgG-Antikörper mehrere Jahre, eventuell auch lebenslang persistieren können (bei 80% der Patienten nach einer Yersinien-Infektion). Bei der chronischen Yersinien-Infektion und der Yersinien-induzierten reaktiven Arthritis können über Jahre, neben Yersinia-spezifischen Antikörper der IgG-Klasse auch persistierende IgA-Antikörper nachgewiesen werden. Die Yersinien-spezifischen Antikörper der IgM-Klasse sind bei Folgeerkrankungen meistens nicht mehr nachweisbar.

Die herkömmlichen serologischen Nachweismethoden, wie die gegen Ganzzellsysate gerichtete Widal- und Komplementbindungsreaktion, besitzen aufgrund der Kreuzreaktivität mit einer Vielzahl von humanen Krankheitserregern (wie z. B. *Bartonella henselae, Borrelia burgdorferi, Chlamydia pneumoniae, Rickettsia rickettsii, Escherichia coli, Brucella* spp., *Salmonella* spp.) nur eine geringe diagnostische Sensitivität und Spezifität. Daher werden heute Enzymimmunoassays (ELISA) und Immunoblots zum Nachweis von IgG-, IgA- und IgM-Antikörpern gegen rekombinant hergestellte virulenzassoziierte Yersinien-spezifische Antigene (z. B. Yops und V-AG) bevorzugt.

Die gegenwärtige Yersinien-Serodiagnostik basiert hauptsächlich auf der Nachweisreaktion der IgG- und IgA- (bedingt auch IgM-) Antwort gegen Virulenzplasmid pYV sezernierte Yop-Proteine, wie z. B. YopD, YopH, YopM, YopE, V-AG und YopN. Die Spezifität und Sensitivität dieser Yop-Antigene ist jedoch verbesserungswürdig und wird erfindungsgemäß durch zusätzliche Yersinien-spezifische Antigene ergänzt.

Zusätzlich bedingt durch die Ähnlichkeit der Ätiologie und des Infektionsvorgangs, einen subklinischen Infektionsverlauf bzw. eine unspezifische Symptomatik sowie eine hohe Durchseuchungsrate, ist die Unterscheidung zwischen *Y. enterocolitica* und *Y. pseudotuberculosis* -Infektionen bzw. durch diese Erreger hergerufene Folgeerkrankungen (wie z. B. ReA bzw. postenteritische Arthritiden, Myokarditis, Glomerulonephritis, Lymphadenopathien, Splenomegalien, *Erythema nodosum*) mit den herkömmlichen diagnostischen Testverfahren schwierig. Ein spezifisches serologisches Nachweisverfahren (d. h. insbesondere Differenzierung zwischen den häufigsten humanpathogenen Yersinien-Arten *Y. enterocolitica* und *Y. pseudotuberculosis*) ist jedoch erforderlich, um eine frühzeitige und effektive Behandlung der Yersiniosen bzw. eine Vorbeugung der Folgeerkrankungen gewährleisten zu können (d. h. adäquate Antibiotikatherapie). Hier können die unterschiedlichen Proteine von *Y. enterocolitica* (MyfA) und *Y. pseudotuberculosis* (PsaA) einen Beitrag leisten.

Gegenstand der vorliegenden Erfindung ist eine Vorrichtung im weiteren Sinne zur serologischen Unterscheidung einer Infektion mit *Yersinia enterocolitica* von einer Infektion mit *Yersinia pseudotuberculosis.* Eine derartige Vorrichtung enthält wenigstens ein Antigen ausgewählt aus einer Gruppe von Antigenen, die den äußeren Oberflächenproteinen oder sezernierte Proteine von Yersinia zugerechnet werden können. In der Vorrichtung muss wenigstens eines dieser Antigene eingesetzt werden, wobei es nicht zwangsläufig erforderlich ist, dass das komplette Protein eingesetzt wird, sondern es kann durchaus genügen, Proteinfragmente einzusetzen, die ein diagnostisch relevantes Epitop aufweisen.

Ausreichend ist ein Fragment eines der nachfolgend aufgezählten Antigene, das mindestens acht aufeinanderfolgende Aminosäuren, bevorzugt mindestens 12, noch bevorzugter mindestens 20, weiter bevorzugt wenigstens 30 aufeinanderfolgende Aminosäuren und ganz besonders bevorzugt wenigstens 50 aufeinanderfolgende Aminosäuren aufweist. Bei der Auswahl der Fragmente wird ein solcher Bereich ausgewählt, der bevorzugt wenigstens ein diagnostisch relevantes Epitop enthält. Die Lokalisierung der Epitop-Bereiche kann durch dem Fachmann bekannte Standardmethoden erfolgen. Es ist möglich, die Hydrophilizität/Hydrophobizität des Proteins mit geeigneten Computerprogrammen zu bestimmen. Hydrophile Bereiche sind in der Regel dazu prädestiniert geeignete Epitope zu tragen, da die hydrophilen Bereiche im gefalteten Protein an der Oberfläche zu liegen kommen. Hydrophobe Bereiche werden eher im Inneren des gefalteten Proteins lokalisiert sein und sind daher an diagnostisch relevanten Epitopen eher nicht beteiligt.

Wenn geeignete Bereiche identifiziert wurden, können diese entweder durch chemische Synthese synthetisiert werden oder rekombinant hergestellt werden. Diese kurzen Peptide können dann mit geeigneten Seren, deren Ätiologie mit anderen, medizinischen Parametern bestimmte wurde, umgesetzt werden. Auf diese Art und Weise kann der Fachmann geeignete Epitope lokalisieren.

Die erfindungsgemäßen Vorrichtungen enthalten also wenigstens ein Antigen bzw. ein Fragment eines dieser Antigene ausgewählt aus der Gruppe bestehend aus Antigenen, die nachfolgend aufgezählt sind. Es handelt sich hierbei um folgende Antigene:

### YopD (Seq ID No. 1)

MTINIKTDSPIITTGSQIDAITTETVGQSGEVKKTEDTRHEAQAIKSSEASLSRSQVPELIKPSQ
GINVALLSKSQGDLNGTLSILLLLLELARKAREMGLQQRDIENKAAITAQKEQVAEMVSGAKL
MIAMAVVSGIMAATSTVASAFSIAKEVKIVKQEQILNSNIAGRDQLIDTKLQQMSNTSDKAVS
REDIGRIWKPEQVADQNKLALLDKEFRMTDSKANAFNAATQPLGQMANSAIQVHRGYSQAE
VKEKEVNASIAANEKQKAEEAMNYNDNFMKDVLRLIEQYVSSHTHAMKAAFGVV.

### YopH (Seq ID No. 2)

MNLSLSDLHRQVSRLVQQESGDCTGKLRGNVAANKETTFQGLTIASGARESEKVFAQTVLS
HVANIVLTQEDTAKLLQSTVKHNLNNYELRSVGNGNSVLVSLRSDQMTLQDAKVLLEAALR
QESGARGHVSSHSHSVLHAPGTPVREGLRSHLDPRTPPLPPRERPHTSGHHGAGEARATA
PSTVSPYGPEARAELSSRLTTLRNTLAPATNDPRYLQACGGEKLNRFRDIQCCRQTAVRAD
LNANYIQVGNTRTIACQYPLQSQLESHFRMLAENRTPVLAVLASSSEIANQRFGMPDYFRQS
GTYGSITVESKMTQQVGLGDGIMADMYTLTIREAGQKTISVPVVHVGNWPDQTAVSSEVTK
ALASLVDQTAETKRNMYESKGSSAVADDSKLRPVIHCRAGVGRTAQLIGAMCMNDSRNSQ
LSVEDMVSQMRVQRNGIMVQKDEQLDVLIKLAEGQGRPLLNS.

### YopN (Seq ID No. 3)

MTTLHNISYGNTTLRNEHPETASSQIVNQTLGQFRGESVQIVSGTLQSIADMAEEVTFVFSE
RKELSLDKRKLSDSQARVSDVEEQVNQYLSKVPELEQKQNVSELLSLLSNSPNISLSQLKAY
LEGKSEEPSEQFKMLCGLRDALKGRPELAHLSHLVEQALVSMAEEQGEAIVLGARITPEAYR
ESQSSVNPLQPLRDTYRDAVMGYQGIYAIWSDLQKRFPNGDIDSVILFLQKALSADLQSQQS
GSGREKLGIVISDLQKLKEFGSVSDQVKGFWQFFSEGKTNGVRPF.

### YopE (Seq ID No. 4)

MPKISSFISTSLPLPTSVSGSSSVGEMSGRSVSQQKSEQYANNLAGRTESPQGSSLASRITE
KLSSMARSAIEFIKRMFSEGSHKPVVTPAPTPAQMPSPTSFSDSIKQLAAETLPKYIQQLSSL
DAETLQKNHDQFATGSGPLRGSITQCQGLMQFCGGELQAEASAILNTPVCGIPFSQWGTIG
GAASAYVASGVDLTQAANELKGLAQQMHQLLSLM.

### YopM (Seq ID No. 5)

MFINPRNVSNTFLQEPLRHSSDLTEIPVEAENVKSKTEYYNAWSEWERNAPPGNGEQREM
AVSRLRDCLDRQAHELELNNLGLSSLPELPPHLERLVASCNSLTELPELPQSLKSLEVYENN
LKALPDLPPLLVDLRVFNNQLEELPELQNLPFLTEIYANNNSLKTLPDLPPSLVDLNVRENYLT
ALPELPQSLIFLDISDNILSGLSELPPNLSCLDASRNGIRSLCDLPPSLVYLDVRDNQLIELPAL
PSGLERLIASFNHLAELPELPPNLYYLDASRNEISSLCDLPPSLVDLNVRKNQLIELPALPPDL
ERLIASFNHLAELPELPPNLSYLDASRNEISSLCDLPPSLVDLNVRKNQLIELPALPPDLERLIA
SFNHLAELPELPPNLSYLDASRNEISSLCDLPPSLVELDVRDNQLIELPALPPHLERLIASLNH
LAEVPELPQNLKQLHVEHNALREFPDIPESVEDLRMDSERVIDPYEFAHETIDKLEDDVFE.

### V- AG (auch LCRV genannt) (Seq ID No. 6)

MIRAYEQNPQHFIEDLEKVRVEQLTGHGSSVLEELVQLVKDKKIDISIKYDPKKDSEVFAERVI
TDDIELLKKILAYFLPEDAILKGGHYDNQLQNGIKRVKEFLESSPNTQWELRAFMAVMHFSLT
ADRIDDDILKVIVDSMNHHGDARSKLREELAELTAELKIYSVIQAEINKHLSSSGTINIHEKSINL
MDKNLYGYTDEEIFKASAEYKILKKMPQTTIKDDELHEVGVIAGAEKQIVSIKNFLESENKRTG
ALGNLKDSYSYNKDNNELSHFATACSDKSRPLNDLVSQKTTQLSDITSRFNSAIEALNRFIQK
YDSVMQRLLDDTR.

Zusätzlich zu dem Antigen aus der ersten Gruppe von Antigenen weist die erfindungsgemäße Vorrichtung weiterhin wenigstens eines von zwei weiteren Proteinen, nämlich entweder das Protein MyfA und/oder das Protein PsaA oder Fragmente eines dieser beiden Proteine auf. Auch hier weisen die Fragmente eine Mindestgröße von wenigstens 8 aufeinanderfolgenden Aminosäuren, bevorzugt wenigstens 12 aufeinanderfolgenden Aminosäuren, noch bevorzugter wenigstens 20, besonders bevorzugt wenigstens 30 und ganz besonders bevorzugt wenigstens 50 aufeinanderfolgenden Aminosäuren eines der Proteine MyfA und/oder PsaA auf.

Es ist bevorzugt, dass die Vorrichtung die beiden vollständigen Proteine MyfA und PsaA oder Fragmente davon zusammen aufweist. Die Aminosäuresequenzen der beiden Proteine MyfA und PsaA (ohne Leader-Sequenz) sind nachfolgend wiedergegeben.

### MyfA (132-AA) (Seq ID No. 7)

MEPTVINSKDISATKTVKEGGSFSVEFKATENEIVSGKLDADTPAFHLVMSDSGEHKGWNVR
PTGASEGGQMVSADGTRVDLHTNELSWDNDHWWIDDGSERVEATFFLAAGDEVKAGEYQF
TGRVEEYVE.

### PsaA (134-AA) (Seq ID No. 8)

MSTVINSKDVSGEVTVKQGNTFHVDFAPNTGEIFAGKQPGDVTMFTLTMGDTAPHGGWRLI
PTGDSKGGYMISADGDYVGLYSYMMSWVGIDNNWYINDDSPKDIKDHLYVKAGTVLKPTTY
KFTGRVEEYVF.

Die Proteine MyfA bzw. PsaA werden durch die nachfolgend wiedergegebenen Nucleotidsequenzen *myfA* bzw. *psaA* codiert und können unter Verwendung geeigneter Vektoren und Wirtszellen rekombinant hergestellt werden.

### myfA (Seq ID No. 9)

atggaaccgactgttattaatagtaaagacatctctgcaacaaaaactgttaaagagggaggttcgttctcagttgaattcaaggc
cactgaaaacgagattgtgtcaggcaaattggatgcagatacacctgccttccatctggtaatgtcggactcaggggaacataa
aggttggaatgttcggcctaccggtgcatctgagggaggacagatggtttctgcagatggtaccagagttgacttacatacaaat
gagctatcgtgggataacgaccactggtggatagatgacggttctgagcgtgtggaagcgactttctttcttgctgctggcgacga
ggttaaagcaggtgaatatcagttcactgggcgtgttgaggaatatgtcgagtaa

### psaA (Seq ID No. 10)

atgtctactgtcattaactccaaggatgtttctggtgaggtgactgtcaagcagggaaacacattccacgtcgattttgcgcctaac
acaggagagatttttgcgggtaaacagccgggtgatgtcactatgtttacgctaactatgggtgatactgcaccacacggtggttg
gcgtttgattccaacaggggactcaaaaggtggatatatgatcagcgccgatggtgactatgttggtttatacagttatatgatgtca
tgggtaggtatagataataactggtatataaatgatgactctcctaaagatataaaagatcatctgtacgttaaggcagggactgtc
cttaaaccaacgacttataaattcacggggcgtgttgaagagtatgtatttt
aa

Bei einer Vorrichtung zum serologischen Nachweis einer Infektion von *Yersinia* Species handelt es sich erfindungsgemäß in bevorzugter Ausführungsform um einen diagnostischen Kit. Darunter versteht man eine Vorrichtung, die von diagnostisch tätigen Labors zur serologischen Diagnose eingesetzt werden. In bevorzugter Ausführungsform handelt es sich hierbei um diagnostische Kits, wobei ELISA-Tests eine bevorzugte Ausführungsform darstellen. Bei den ELISA-Testkits wird üblicherweise das Antigen an die Mikrotiterplatten gebunden, das Patientenserum kann mit den Antigenen reagieren und gebundene Antikörper aus dem Serum gegen die Antigene werden meist mit Anti-Antikörpern, die eine Markierung, vorzugsweise eine Enzym-Markierung, tragen, nachgewiesen.

Eine andere bevorzugte Ausführungsform der erfindungsgemäßen Vorrichtung sind sogenannte Line-Tests. Hierbei werden mehrere Antigene nach einem vorbestimmten Muster auf die Teststreifen aufgebracht. Die zu untersuchenden Seren werden mit den Teststreifen umgesetzt und Antigen-Antikörper Reaktionen werden durch Enzym-markierten Antikörper und anschließende Färbereaktion nachgewiesen. Aus dem spezifischen Muster der Reaktivitäten bzw. Farbsignale kann auf die Infektion geschlossen werden.

Bei der erfindungsgemäßen Vorrichtung kann es sich auch um einen Immunoblot bzw. Western Blot handeln. Hierbei werden die diagnostisch relevanten Proteine zunächst der Größe nach beispielsweise durch Diffusion, Kapillarwirkung oder Elektrophorese aufgetrennt und auf ein Trägermaterial, beispielsweise eine Nylonmembran oder eine Nitrocellulosemembran übertragen und dort fixiert. Dieses Trägermaterial mit den daran gebundenen Proteinen bzw. Proteinfragmenten wird mit Serum von Patienten umgesetzt. Die darin enthaltenen Antikörper (vor allem IgG, IgM, IgA) binden an die Antigene und werden beispielsweise durch Reaktion mit einem Anti-Antikörper nachgewiesen. Diese Anti-Antikörper tragen in der Regel eine Markerung. Hierbei kann es sich um ein Enzym handeln, das eine Farbreaktion katalysiert, es kann sich aber auch um fluoreszierende Reste oder radioaktive Reste handeln. Wichtig ist, dass mit den Anti-Antikörpern an Antigen gebundene Antikörper nachgewiesen werden können.

In einer anderen Ausführungsform handelt es sich bei der erfindungsgemäßen Vorrichtung um einen auf Kügelchen basierenden Test (sog. Bead-Based-Assay). Eine bekannte kommerzielle Anwendungsform von diesen sphärischen Mikroarrays stellt die Luminex-XMAB-Technologie von Luminex Corporation (Austin, USA) dar. Bei diesem System werden Mikrokugeln eingesetzt und mit Hilfe der Flow-Cytometrie ausgewertet. Bei Fluoreszenzmarkierten Ausführungsformen können die erfindungsgemäßen Antigene auf Kugeln fixiert werden und die Bindung der Antikörper an die Antigene wird durch geeignete Markierungen, z.B. Fluoreszenz sichtbar gemacht.

Eine weitere bevorzugte Ausführungsform der erfindungsgemäßen Vorrichtung sind Protein-Mikroarrays. Hierbei werden verschiedene Antigene auf engem Raum an eine Oberfläche fixiert. Da bekannt ist, welches Antigen an welcher Stelle vorhanden ist, kann nach Sichtbarmachung der Antigen-Antikörperreaktion beispielsweise mit Hilfe einer Farbreaktion oder Fluoreszenz-Markierung auch eine Aussage getroffen werden, welches Antigen mit den Antikörpern in dem Serum reagiert hat.

Üblicherweise werden die einzelnen Antigene so in der Vorrichtung fixiert, dass nach der Reaktion unterscheidbar festgestellt werden kann, mit welchem Antigen die in dem Serum vorhandenen Antikörper reagiert haben. Bei einem ELISA-Test werden beispielsweise die einzelnen Antigene in verschiedene Kavitäten der Mikrotiterplatte eingebracht. Beim Line-Assay werden die Antigene auf verschiedene Streifen des Trägermaterials aufgesprüht und bei den sphärischen und planaren Mikroarrays werden die jeweiligen Antigene immer an definierter Stelle auf den Träger bzw. definierten Beads aufgebracht. Die erfindungsgemäßen Vorrichtungen dienen dem Nachweis von humanpathogenen *Yersinia-*Species bzw. Subspecies. Durch die Kombination von verschiedenen Antigenen ist es einerseits möglich, einen sensitiven und spezifischen Nachweis der Erreger zu führen und andererseits ist in bevorzugter Ausführungsform auch ein differentialdiagnositischer Nachweis möglich, wobei zwischen einer Infektion verursacht durch *Yersinia enterocolitica* unterscheiden werden kann von einer Infektion mit *Yersinia pseudotuberculosis.*

Ein anderer wesentlicher Aspekt der vorliegenden Erfindung ist auch, dass ein serologischer Nachweis bzw. eine serologische Differenzierung dann möglich ist, wenn die akute Yersinieninfektion bereits abgeklungen ist. Für die Therapie von derartigen Folgeerkrankungen ist es wesentlich, dass unterscheiden werden kann, durch welches Bakterium (*Yersinia enterocolitica* oder *Yersinia pseudotuberculosis*) die ursprüngliche Infektion verursacht wurde.

Die erfindungsgemäße Vorrichtung weist also wenigstens zwei verschiedene Antigene auf, wobei das eine Antigen ausgewählt ist aus der Gruppe bestehend aus den oben näher dargestellten Antigenen YopD, YopH, YopM, YopE, V-AG und YopN. Die andere Gruppe umfasst die Antigene MyfA und/oder PsaA.

In besonders bevorzugter Ausführungsform beinhaltet die Vorrichtung das Antigen YopD sowie das Antigen YopH zusammen mit entweder MyfA und/oder PsaA.

In einer noch weiter bevorzugten Ausführungsform beinhaltet die erfindungsgemäße Vorrichtung die Antigene YopD, YopH, YopM und entweder MyfA oder PsaA, wobei aber der kombinierte Einsatz von MyfA und PsaA ganz besonders bevorzugt ist.

In besonders bevorzugter Ausführungsform weist also die erfindungsgemäße Vorrichtung die Proteine MyfA und/oder PsaA oder Fragmente hiervon, wie vorstehend definiert, zusammen auf mit dem folgenden Protein bzw. Proteinkombinationen, wobei diese Proteine auch in Form von Fragmenten, wie vorstehend definiert vorliegen können. Die erfindungsgemäße Vorrichtung weist wenigstens das Protein YopD auf, das bevorzugt zusammen mit YopH und noch bevorzugter zusammen mit YopM kombiniert mit MyfA und/oder PsaA eingesetzt wird.

Eine weiter bevorzugte Ausführungsform beinhaltet die Proteine oder Fragmente von YopD, YopH, YopM und YopE zusammen mit MyfA und/oder PsaA.

Eine andere bevorzugte Ausführungsform beinhaltet die Proteine und/oder Fragmente von YopD, YopH, YopM, YopE, V-AG zusammen mit MyfA und/oder PsaA.

Eine weitere bevorzugte Ausführungsform beinhaltet eine Kombination von Proteinen und/oder Fragmenten der folgenden Bezeichnungen: YopD, YopH, YopM, YopE, V-AG, YopN, sowie MyfA und/oder PsaA.

Wenn erfindungsgemäß Fragmente der Antigene eingesetzt werden, ist bei dem Antigen MyfA einerseits das N-terminale Fragment mit den Aminosäuren 30-41 und andererseits das C-terminale Fragment mit den Aminosäuren 148-159 besonders bevorzugt. In dem PsaA Protein sind die Fragmente besonders bevorzugt, die die Aminosäuren 27-37 (N-Terminus) sowie 144-155 (C-Terminus) beinhalten. In diesem Fall werden Fragmente eingesetzt, die diese Aminosäuresequenz beinhalten und die oben näher definierten Größen aufweisen.

Gegenstand der vorliegenden Erfindung ist also die Verwendung eines antigenen Proteins MyfA oder eines Fragments hiervon, wie oben näher definiert, entweder allein oder zusammen mit dem antigenen Protein PsaA oder einem Fragment hiervon, wie oben definiert, zum serologischen Nachweis einer durch *Yersinia* verursachten Infektion. Diese Unterscheidung hat klinische Relevanz, weil eine Infektion, verursacht durch *Yersinia enterocolitica,* anders behandelt werden kann, als eine Infektion hervorgerufen durch *Yersinia pseudotuberculosis.*

Mit der erfindungsgemäßen Vorrichtung können Yersinien nachgewiesen werden, die eine erhebliche Bedeutung haben, da sie humanpathogen sind. Die Yersinien kommen zum Teil in Nutztieren vor und können bei einer Infektion eine Erkrankung am Menschen hervorrufen. Es kann daher eine erhebliche medizinische und epidemiologische Bedeutung haben, mit Hilfe eines einfachen und verhältnismäßig kostengünstigen Testkits nachzuweisen, wie die Infektionsketten verlaufen sind. Dadurch sind Rückschlüsse auf die Quellen der Krankheitserreger möglich und diese können identifiziert und gegebenenfalls unschädlich gemacht werden. Es ist also möglich, die Erreger von Yersiniosen nachzuweisen und zu unterscheiden.

Die Figuren verdeutlichen bevorzugte Ausführungsformen der vorliegenden Erfindung und erläutern die in den Beispielen erhaltenen Ergebnisse.
Figur 1 zeigt die homologen Aminosäuresequenzbereiche der MyfA- und PsaA-Antigene. Die homologen Bereiche (Konsensus) sind schwarz markiert. Die N- und C-terminalen homologen Bereiche sind umrandet.
Figur 2 erläutert die *In silico*-Bestimmung der antigenen Domänen von MyfA und PsaA. Der Antigenitätsindex wurde anhand des Jameson-Wolf -Algorithmus und die Hydrophilizität des Antigens anhand des Kyte-Doolittle -Algorithmus berechnet. Die sog. Leader-Sequenz ist mit einem schwarzen Pfeil markiert.
Figur 3 zeigt eine schematische Darstellung der DNA-Sequenzen der vier verwendeten His-Tag-MyfA-Fusionsproteine. Die zur Amplifikation verwendeten Primersequenzen sind mit Pfeilen (I, II, III, IV) markiert. Die Leader-Sequenzen sind hellgrau und die mit PsaAhomologen Bereichen weiß dargestellt.
Figur 4 zeigt die chromatographische Trennung (SDS-Polyacrylamid-Gelelektrophorese) der MyfA-Teilfragmente *myfA* 1-441, *myfA* 121-441 und *myfA* 121-447 (Fig. 3) mit anschließender Coomassie-Blau-Färbung (links) und Immunoblot (*myfA, myfA* 1-441, *myfA* 121-441 und *myfA* 121-447; Fig. 3) mit Anti-*Y*. *enterocolitica* (O:3 bzw. O:9)- und Anti-MyfA - Seren aus Kaninchen (rechts).
Figur 5 zeigt die Überprüfung der serologischen Reaktivität des aufgereinigten MyfA-Gesamtproteins (MyfA) und der aufgereinigten MyfA -Teilfragmente MyfA 1-441, MyfA 121-441 und MyfA 121-447 (Fig. 1; Fig. 3) mittels Line-Assays. Die Teststreifen wurden mit vier verschiedenen Anti-*Y. enterocolitica* O:3, O:8 bzw. O:9 -Seren aus Kaninchen (1-4) vor (*) und nach der Infektion inkubiert.

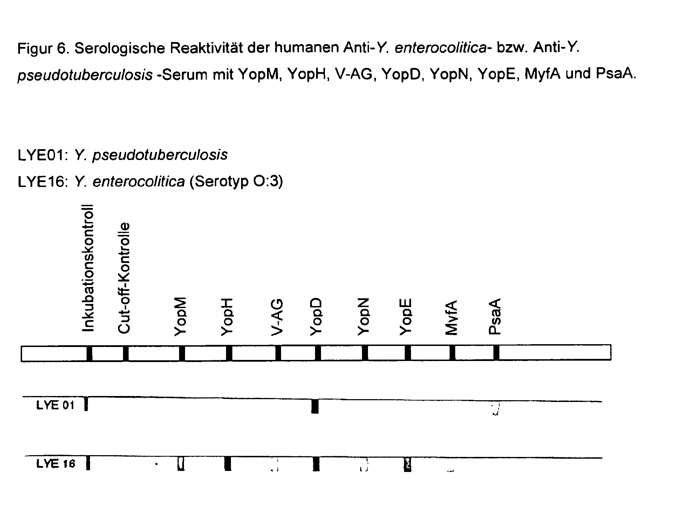

Figur 6 zeigt die Serologische Reaktivität der humanen Anti-*Y. enterocolitica-* bzw. Anti-*Y. pseudotuberculosis* -Serum mit YopM, YopH, V-AG, YopD, YopN, YopE, MyfA und PsaA im Line-Assay
Figur 7 stellt die IgG-Antwort gegen Yop-, MyfA-MIK- und PsaA-MIK-Antigene unter den getesteten bayerischen Blutspenderseren (n=40) dar.
Figur 8 erläutert die IgG-Antwort gegen Yop-, MyfA-MIK- und PsaA-MIK-Antigene unter den getesteten Yersiniose-Patientenseren aus Finnland (n=18).
Figur 9 zeigt die IgG-Antwort gegen Yop-, MyfA-MIK- und PsaA-MIK-Antigene unter den getesteten Yersiniose-Patientenseren aus Deutschland (n=23). Unter dem Oberflächenprotein ist YopD für den Einsatz in diagnostischen Testvorrichtungen besonders geeignet.

### Beispiel 1

### In silico Bestimmung der immunogenen Domänen der MyfA- und PsaA-Antigene

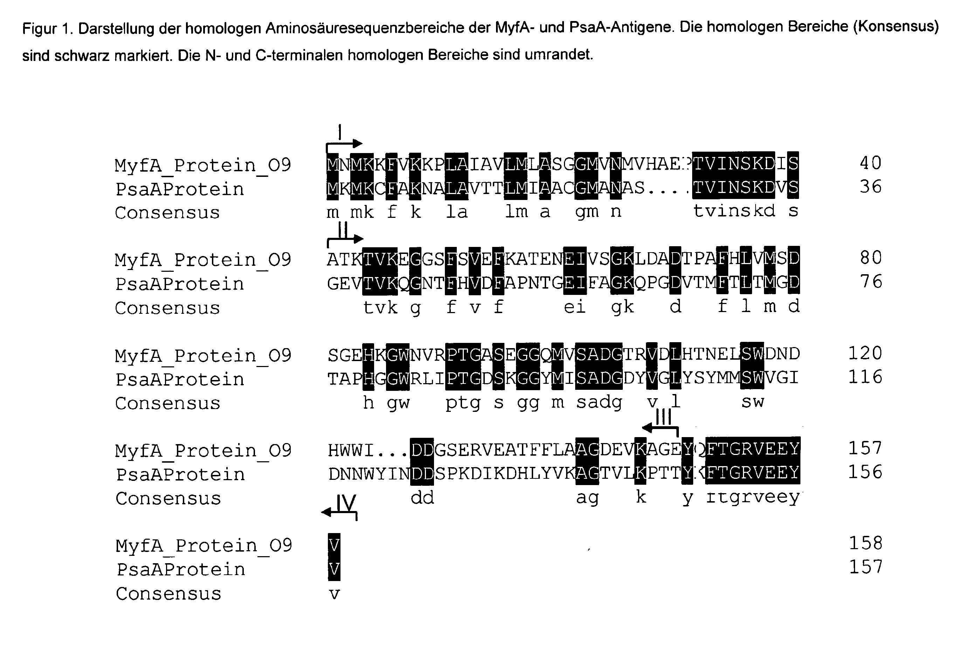

Die Bestimmung der homologen Antigenbereiche, die für die immunogene Reaktivität bzw. Kreuzreaktivität der MyfA- und PsaA- Antigene verantwortlich sein könnten, erfolgte *in silico* mittels direkten Aminosäuresequenzvergleichs (siehe Figur 1). Die homologen Bereiche befinden sich hauptsächlich im N- und C-Terminus des MyfA- (AA 32-40 und 150-159) und PsaA- (AA 29-38 und 149-158) Antigens.

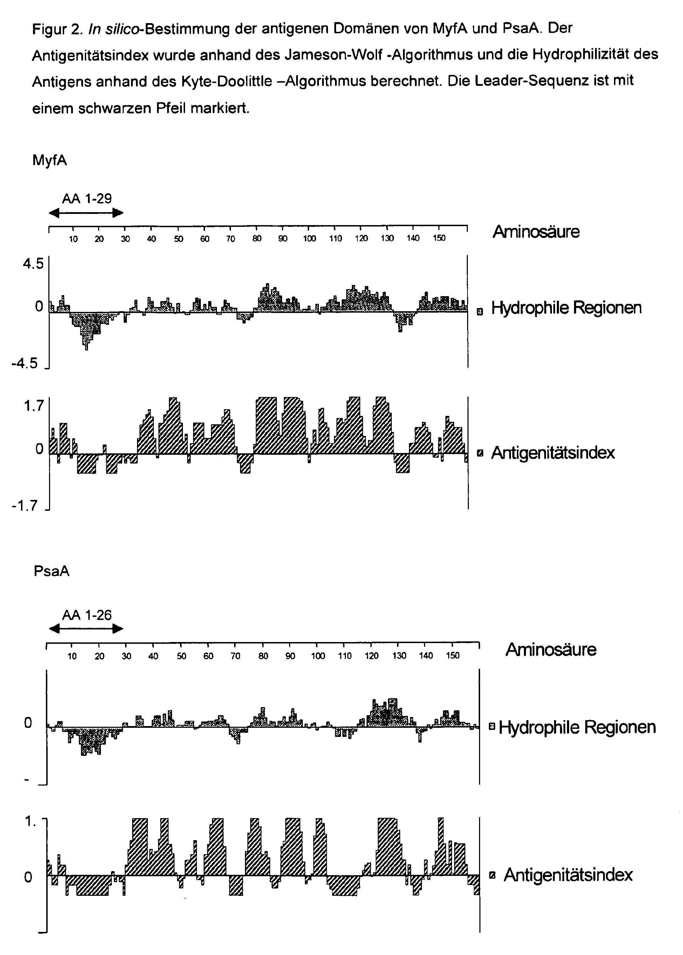

Der Antigenitätsindex und der hydrophile Charakter der Antigenregionen wurde *in silico* anhand der Algorithmen von Jameson und Wolf (Comput. Appl. Biosci. (1988) S. 181-186) bzw. von Kyte und Doolittle (Kyte and Doolittle, (1982) J. Mol. Biol., 157, S. 105-132) bestimmt (Fig. 2). Die acht > 5 AA immunogenen Domänen des PsaA-Antigens befinden sich voraussichtlich in AA 30-48, 52-56, 59-67, 74-83, 87-96, 122-133, 144-150 und 151-158. Die sieben putativen > 5 AA immunogenen Domänen des MyfA-Antigens wurden in AA 35-42, 43-54, 55-73, 79-100, 101-133, 139-148 und 152-159 lokalisiert. Diese Fragmente werden erfindungsgemäß bevorzugt in den diagnostischen Tests eingesetzt. Da jedoch der prädikative Kyte-Doolittle-Algorithmus nur bedingt aussagekräftig ist, wurden die immunogenen Epitope der neuen Antigene experimentell bestimmt (Beispiel 2).

### Vollständige Gesamtprotein-Sequenzen:

### MyfA (159-AA) (Seq ID No. 11)

AA 1-29: Leader-Sequenz
AA 30-41: **N-terminales Fragment** (Beispiel 2)
AA 148-159: **C-terminales Fragment** (Beispiel 2)

MNMKKFVKKPLAIAVLMLASGGMVNMVHAEPTVINSKDISATKTVKEGGSFSVEFKATENEI
VSGKLDADTPAFHLVMSDSGEHKGWNVRPTGASEGGQMVSADGTRVDLHTNELSWDND
HWWIDDGSERVEATFFLAAGDEVKAGEYQFTGRVEEYVE

### PsaA (158-AA) (Seq ID No. 12)

### AA 1-26: Leader-Sequenz

MKMKCFAKNALAVTTLMIAACGMANASTVINSKDVSGEVTVKQGNTFHVDFAPNTGEIFAG
KQPGDVTMFTLTMGDTAPHGGWRLIPTGDSKGGYMISADGDYVGLYSYMMSWVGIDNNW
YINDDSPKDIKDHLYVKAGTVLKPTTYKFTGRVEEYVF

### Beispiel 2

### Experimentelle Bestimmung der immunogenen Domänen der MyfA-Antigene

### Herstellung der MyfA-Teilfragmente

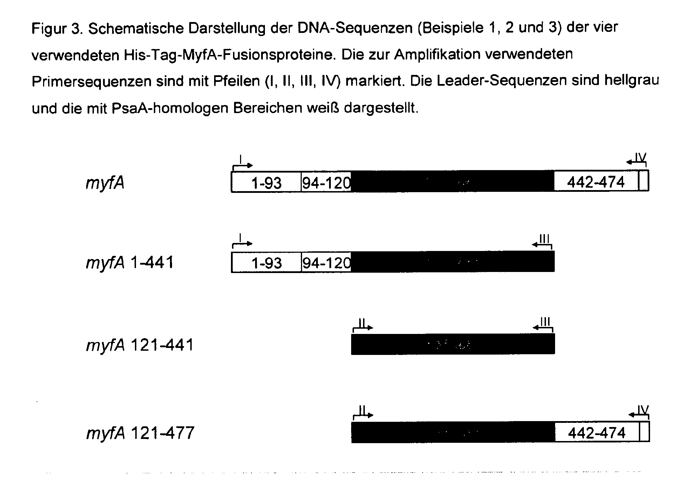

Ausgehend vom vollständigen Leserahmen des MyfA-Antigens (Beispiel 1) wurden vier Fragmente hergestellt. Dabei wurden sowohl der gesamte Leserahmen einschließlich des Leader-Peptids als auch N-, C, und N- und C-terminal verkürzte Teilfragmente dargestellt (Fig. 1; Fig. 3).

Die spezifische Amplifikation der *myfA*-Teilfragmente erfolgte mittels PCR mit chromosomaler DNA aus *Y. enterocolitica* Serotyp O:3/4 *(Y. enterocolitica* subsp. *palearctica* Stamm Y-11; DMSZ 13030) als Template.

Die resultierenden Amplifikate wurden mittels Restriktionsendonukleasen enzymatisch gespalten und in einen geeigneten Vektor, pET21-b, der mit denselben Restriktionsendonukleasen wie die Amplifikate geschnitten wurde, ligiert. Nach der Transformation des Ligationsansatzes in einen geeigneten *E. coli*-Stamm, z. B. BL21 pLys, wurden einzelne Klone auf das Vorhandensein von *myfA* und *psaA* mittels Agarose-Gelelektrophorese enzymatisch gespaltener Plasmid-DNA getestet und anschließend sequenziert. Des Weiteren wurde die Expression der Teilfragmente durch Analyse der Expressionsprodukte mittels SDS-Polyacrylamid-Gelelektrophorese mit anschließender Coomassie-Blau-Färbung bzw. anschließendem Transfer auf Nitrozellulose gefolgt von einer immunologischen Detektion identifiziert und charakterisiert. Die Reinigung der Fusionsproteine erfolgte mittels Ni-NTA-Säulenchromatographie gemäß Herstellerangaben (Firma Qiagen).

Neben dem kompletten Leserahmen von MyfA (MyfA; Fig. 3) wurden drei Teilfragmente des Antigens als His-Tag-Fusionsproteine in Vektor pET21-b kloniert. Das erste Fragment (MyfA 1-441) wurde ohne den zu PsaA homologen C-terminalen Bereich (Beispiel 1; Fig. 1) kloniert und umfasst die AA 1-147 (Nukleotid [NT] 1-441). Das zweite Fragment (MyfA 121-441) kodiert für die AA 40-147 (NT 121-441) und enthält somit nicht die zu PsaA homologen N-und C-terminalen Bereiche (Beispiel 1; Fig. 1). Das dritte Fragment (MyfA 121-477) kodiert für die AA 40-159 (NT 121-477) und enthält nicht die N-terminale Sequenz von MyfA (Beispiel 1; Fig. 3; Fig. 1).

Nachfolgende Oligonukleotid-Primer wurden zur Darstellung der Sequenzen verwendet.
Primer 1 (I):
   myfA-f: GTA ATT CCA TAT GAA TAT GAA AAA ATT TGT (Seq ID No. 13)
Primer 2 (II):
   myfA_121f: GTA ATC CCA TAT GGC AAC AAA AAC TGT (Seq ID No. 14)
Primer 3 (III):
   myfA_441rev: TTA CTC GAG TTC ACC TGC TTT AAC (Seq ID No. 15)
Primer 4 (IV):
   myfA_rev: ATC TAC TCG AGC TCG ACA TAT TCC TCA A (Seq ID No 16)

Dabei wurden die folgenden Primerkombinationen verwendet:
*myfA*: Primer 1 und Primer 4
*myfA* 1-441: Primer 1 und Primer 3
*myfA* 121-441: Primer 2 und Primer 3
*myfA* 121-477: Primer 2 und Primer 4

### Beispiel 3

### Überprüfung der Antigenität

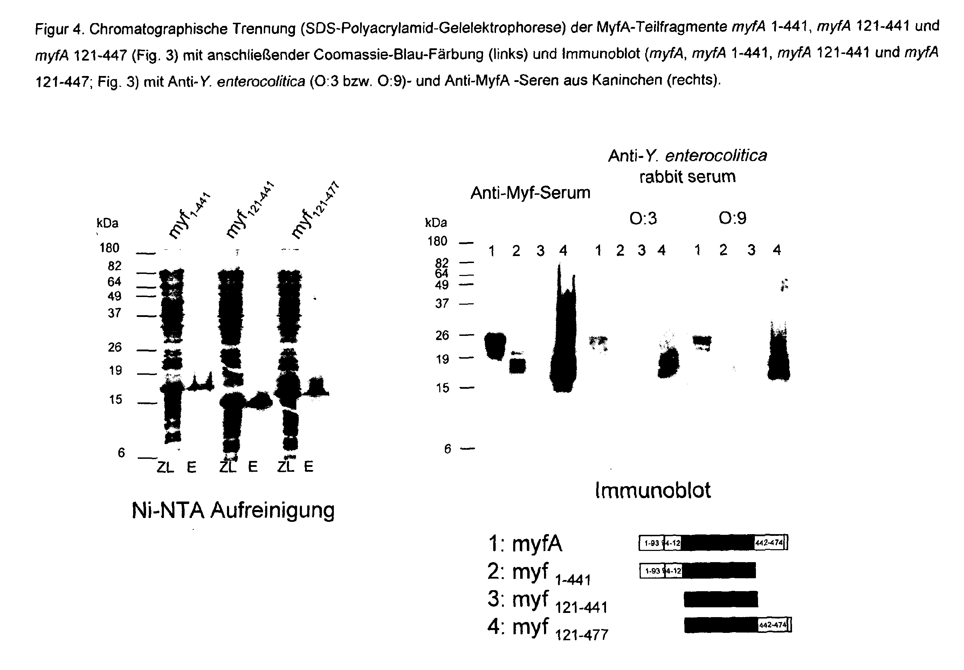

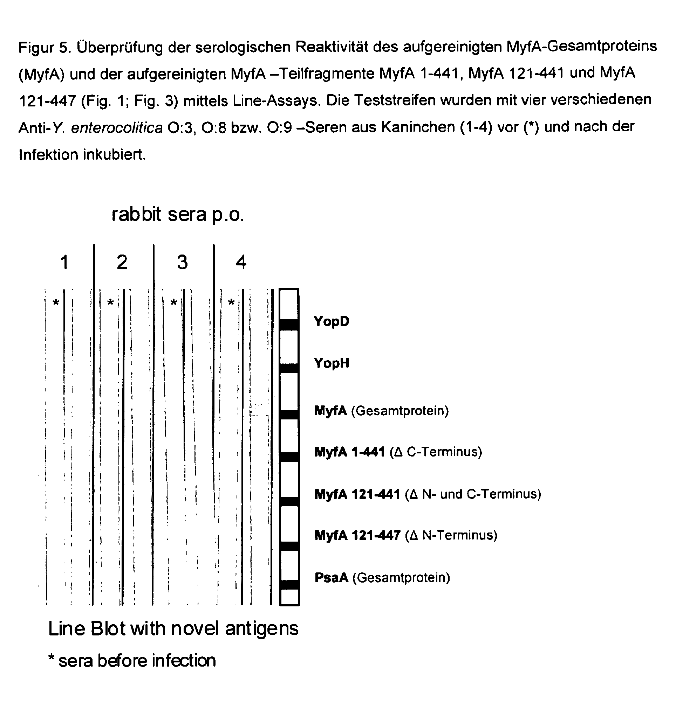

Die immunologische Reaktivität/Antigenität der MyfA-Fusionsproteine (Beispiel 2) wurde mittels Immunoblot (Fig. 4) und Line-Assay getestet (Fig. 5)

In Fig. 4 links sind jeweils Zellysate (ZL) von MyfA-Gesamtprotein (MyfA, inklusiver Leader-Sequenz; Beispiel 1; Fig. 1; Beispiel 2; Fig. 3) beziehungsweise eines der drei MyfA-Teilfragmente (MyfA 1-441, MyfA 121-441 und MyfA 121-447) exprimierenden *E. coli* BL21 pLys -Zellen sowie die mittels Ni-NTA-Säulenchromatographie aufgereinigten His-getaggten rekombinanten MyfA-Proteine (MyfA 1-441, MyfA 121-441 und MyfA 121-447) dargestellt.

Die rechte Darstellung in Fig. 4 zeigt einen Immunoblot mit zwei Anti-*Y*. *enterocolitica* O:3 bzw. O:9 - Seren und einem Anti-MyfA-Serum aus Kaninchen. Das am N- und C-Terminus verkürzte MyfA -Protein (MyfA 121-441; Beispiel 1; Fig. 1; Beispiel 2; Fig. 3) zeigt keine Reaktion mit den verwendeten Seren. Das am C-Terminus verkürzte Protein MyfA 1-441 (Fig. 1; Fig. 2) zeigt eine schwächere Reaktion als das am N-Terminus verkürzte Protein MyfA 121-447 (Fig. 1; Fig. 2). Somit scheint der C-Terminus einen besonders wichtigen immunogenen Bereich zu beinhalten, aber auch der N-Terminus ist diagnostisch bedeutend.

Durch Nachweis des His-tags mit einem Ni-Konjugat aus Kaninchen konnten die Proteine nachgewiesen werden (sehr schwache Reaktion, keine Doppelbanden). Der Immunoblot mit einem Anti-Y. *pseudotuberculosis* -Serum aus Kaninchen zeigte keine Reaktion.

Die Überprüfung der immunologischen Reaktivität/Antigenität des aufgereinigten MyfA-Gesamtproteins (MyfA) und der aufgereinigten MyfA -Teilfragmente MyfA 1-441, MyfA 121-441 und MyfA 121-447 (Beispiel 1, Fig. 1; Beispiel 2; Fig. 3) erfolgte mittels Line-Assays (Fig. 5). Die Teststreifen wurden mit vier verschiedenen Anti-*Y. enterocolitica*-Seren aus Kaninchen (Serum 1 und 4: Serotyp O:3; Serum 2: Serum O:9, Serum 3: O:8) vor (*) und nach der experimentellen peroralen Infektion inkubiert. Als Immunisierungs- bzw. Assay-Kontrolle wurden rekombinant hergestellte YopD- und YopH-Antigene in den Assay eingebaut. Zusätzlich, um die Kreuzreaktivität der MyfA- und PsaA-Antigene evaluieren zu können, wurde auch rekombinant hergestelltes PsaA-Antigen in den Assay eingefügt (Fig. 5). Die Reaktivität der Antigene wurde außerdem mit einem Anti-Y. *pseudotuberculosis* - Serum aus Kaninchen überprüft.

Die vor Infektion abgenommenen Blutproben zeigten keine Reaktivität mit den Antigenen des Assays (d. h. YopD, YopH, MyfA, MyfA 1-441, MyfA 121-441, MyfA 121-447, PsaA). Nach der experimentellen Infektion reagierten alle vier Proben mit den Yersinien-spezifischen Antigenen YopD und YopH (Fig. 5). Wie schon beim Immunoblot dargestellt (Fig. 4), zeigten das C- (Δ C-Terminus) bzw. das N- und C- (Δ N- und C-Terminus) terminal verkürzte MyfA-Protein deutlich verminderte und das N-terminal verkürzte Protein (Δ N-Terminus) verminderte Reaktivität im Vergleich zum MyfA-Gesamtprotein (Fig. 5). Interessanterweise reagierten die Seren Nr. 2 (sehr schwach), 3 (schwach) und 4 (positiv) auch mit dem aufgetragenen PsaA-Antigen. Das Anti-Y. *pseudotuberculosis* -Serum aus Kaninchen reagierte sehr stark mit YopD und PsaA, zeigte aber keine Reaktivität mit dem Gesamtantigen MyfA oder dessen Teilfragmenten. Die in dem Assay auftretende Kreuzreaktivität der Anti-*Y*. *enterocolitica* -Seren mit PsaA wird möglicherweise durch semioptimale Produktionsbedingungen verursacht (d.h. Aufreinigung über His-Taq, Pufferkonditionen oder zu hohe Antigenkonzentration). Für folgende Experimente wurden die MyfA- und PsaA Antigene umkloniert (ohne His-Taq und Leader-Sequenz), aufgereinigt und die Assay-Konditionen optimiert (Beispiele 3-5).

### Beispiel 4

### Herstellung und Reinigung der rekombinanten MyfA- und PsaA-Antigene

Ausgehend von den vollständigen Leserahmen (Beispiel 1) der beiden Antigene wurden MyfA (*pmyfA* MIK)- und PsaA (p*psaA*-MIK)-Expressionsklone hergestellt. Nachfolgende Oligonukleotid-Primer wurden zur Darstellung der Sequenzen verwendet. Die Proteine wurden ohne Leader-Sequenz (MyfA AA 1-29 und PsaA AA 1-26; Beispiel 1) hergestellt, da die vorherigen Experimenten zeigten, dass das Leader-Peptid eine reduzierte Expression hervorruft.

### pmyfA-MIK

Primer 5 (Seq ID No. 17):
   myfA-F-Ndel: CAC ATA TGG AAC CGA CTG TTA TTA ATA GTA AAG ACA TC
Primer 6 (Seq ID No. 18):
   myfA-R-Baml: ATG GAT CCT TAC TCG ACA TAT TCC TCA ACA CG

### ppsaA-MIK

Primer 7 (Seq ID No. 19):
   psaA-F-Ndel: GCC ATA TGT CTA CTG TCA TTA ACT CCA AGG ATG
Primer 8 (Seq ID No. 20):
   psaA-R-Baml: CAG GAT CCT TAA AAT ACA TAC TCT TCA ACA CGC C

Die spezifische Amplifikation des *myfA*-Fragments erfolgte mittels PCR mit chromosomaler DNA aus *Y. enterocolitica* Serotyp O:3/4 *(Y. enterocolitica* subsp. *palearctica* Stamm Y-11; DMSZ 13030) als Template. Das *psaA*-Fragment wurde mit chromosomaler DNA aus *Y*. *pseudotuberculosis* Serotyp 1A amplifiziert.

Die resultierenden Amplifikate wurden mittels Restriktionsendonukleasen *Nde* I und Bam HI enzymatisch gespalten und in einen geeigneten Vektor, pET3c (New England Biolabs) ligiert. Nach der Transformation des Ligationsansatzes in den E. coli-Stamm UT 5600 (Elish et al. [1998] J. Gen. Microbiol., 134, S. 1355-1364) wurden die Klone auf das Vorhandensein der *myfA*- und *psaA* -Fragmente mittels Agarose-Gelelektrophorese von enzymatisch gespaltener Plasmid-DNA und mittels DNA-Sequenzierung (siehe unten) getestet. Des Weiteren wurde die Expression der Antigene durch Analyse der Expressionsprodukte MyfA-MIK und PsaA-MIK mittels SDS-Polyacrylamid-Gelelektrophorese mit anschließender Coomassie-Blau-Färbung bzw. anschließendem Transfer auf Nitrozellulose gefolgt von einer immunologischen Detektion identifiziert und charakterisiert.

Die Reinigung der rekombinanten Proteine MyfA-MIK und PsaA-MIK erfolgte mittels Anionenaustausch- und Kationenaustausch-Säulenchromatographie. Im ersten Schritt wurde ein Anionenaustausch (Q-Sepharose Fast Flow; GE Healthcare, München, Deutschland), im zweiten ein Kationenaustausch (S-Source 15; GE Healthcare) und im dritten ein Anionenaustausch (Q-Source 30; GE Healthcare) durchgeführt. Die einzelnen Reinigungsschritte bzw. das aufgereinigte Protein wurde mittels SDS-Polyacrylamid-Gelelektrophorese mit anschließender Coomassie-Blau-Färbung bzw. anschließendem Transfer auf Nitrozellulose gefolgt von einer immunologischen Detektion überprüft und hinsichtlich des Reinheitsgrads, möglicher Proteinspaltung und immunologischer Reaktivität charakterisiert.

### Beispiel 5

### DNA-Sequenzierung der pmyfA-MIK und ppsaA-MIK-Expressionsklone und daraus resultierende Proteinsequenzen (AA) MyfA-MIK und PsaA-MIK

Die START (**atg**)- und STOP (**uaa**)-Kodons sind schwarz markiert.

### DNA-Sequenzen

### der pmyfA-MIK (396 bp) (Seq ID No. 21):

atggaaccgactgttattaatagtaaagacatctctgcaacaaaaactgttaaagagggaggttcgttctcagttgaattcaaggc
cactgaaaacgagattgtgtcaggcaaattggatgcagatacacctgccttccatctggtaatgtcggactcaggggaacataa
aggttggaatgttcggcctaccggtgcatctgagggaggacagatggtttctgcagatggtaccagagttgacttacatacaaat

gagctatcgtgggataacgaccactggtggatagatgacggttctgagcgtgtggaagcgactttctttcttgctgctggcgacga
ggttaaagcaggtgaatatcagttcactgggcgtgttgaggaatatgtcgagtaa

### ppsaA-MIK (402 bp) (Seq ID No. 22):

atgtctactgtcattaactccaaggatgtttctggtgaggtgactgtcaagcagggaaacacattccacgtcgattttgcgcctaac
acaggagagatttttgcgggtaaacagccgggtgatgtcactatgtttacgctaactatgggtgatactgcaccacacggtggttg
gcgtttgattccaacaggggactcaaaaggtggatatatgatcagcgccgatggtgactatgttggtttatacagttatatgatgtca
tgggtaggtatagataataactggtatataaatgatgactctcctaaagatataaaagatcatctgtacgttaaggcagggactgtc
cttaaaccaacgacttataaattcacggggcgtgttgaagagtatgtattttaa

### Aminosäuresequenzen (AA)

### MyfA-MIK (132-AA) (Seq ID No. 23)

MEPTVINSKDISATKTVKEGGSFSVEFKATENEIVSGKLDADTPAFHLVMSDSGEHKGWNVR
PTGASEGGQMVSADGTRVDLHTNELSWDNDHWWIDDGSERVEATFFLAAGDEVKAGEYQF
TGRVEEYVE.

### PsaA-MIK (134-AA) (Seq ID No. 24)

MSTVINSKDVSGEVTVKQGNTFHVDFAPNTGEIFAGKQPGDVTMFTLTMGDTAPHGGWRLI
PTGDSKGGYMISADGDYVGLYSYMMSWVGIDNNWYINDDSPKDIKDHLYVKAGTVLKPTTY
KFTGRVEEYVF.

### Beispiel 6

### Serologische Differenzierung der Yersiniosen verursacht durch Y. enterocolitica oder Y. pseudotuberculosis

Zwei humane Serumproben wurden mittels Widal-Reaktion als Anti-*Y. enterocolitica* Serotyp O:3 (LYE16)- bzw. als Anti-*Y*. *pseudotuberculosis* (LYE01) -IgG-positiv definiert.

Der Vergleich der serologischen IgG-Reaktivität von Yop-, MyfA-MIK- und PsaA-MIK - Antigenen (Beispiel 3) mittels Line-Assays zeigte, dass die *Y. pseudotuberculosis* -positive Serumprobe sehr stark mit YopD und PsaA reagierte. Die Anti-*Y. enterocolitica*-IgG-positive Probe reagierte hingegen mit den Antigenen YopM, YopH, V-AG, YopD, YopN, YopE und MyfA. Diese Serumprobe zeigte auch eine sehr schwache Reaktivität mit PsaA.

Somit ergibt sich ein Hinweis darauf, dass die Antigene MyfA-MIK und PsaA-MIK die serologische Differenzierung von *Y. enterocolitica* und *Y. pseudotuberculosis* -Infektionen ermöglicht.

### Beispiel 7

### Vergleich der serologischen Prävalenz der Anti-Yop-, Anti-MyfA- und Anti-PsaA-Antikörper der IgG-, IgM- und IgA-Klasse in Serumproben von bayerischen Blutspendern bzw. in Serumproben von finnischen und deutschen Yersiniose-Patienten

Die IgG-, IgM- und IgA-Antwort gegen rekombinant hergestellte YopM-, YopH-, V-AG-, YopD, YopN-, YopE-, MyfA-MIK- und PsaA-MIK- (Beispiel 4 und 5) Antigene mit Seren aus drei verschiedenen Serensammlungen (d. h. bayerische Blutspender n=40; Bayern; Deutschland, Yersiniose-Patienten aus Finnland [KTL, n=18] und Yersiniose-Patienten aus Deutschland [YeD, n=23]) wurden mittels Line-Assays untersucht. Als Referenztest wurde der recomLine Yersinia der Fa. Mikrogen GmbH verwendet.

### Blutspenderseren

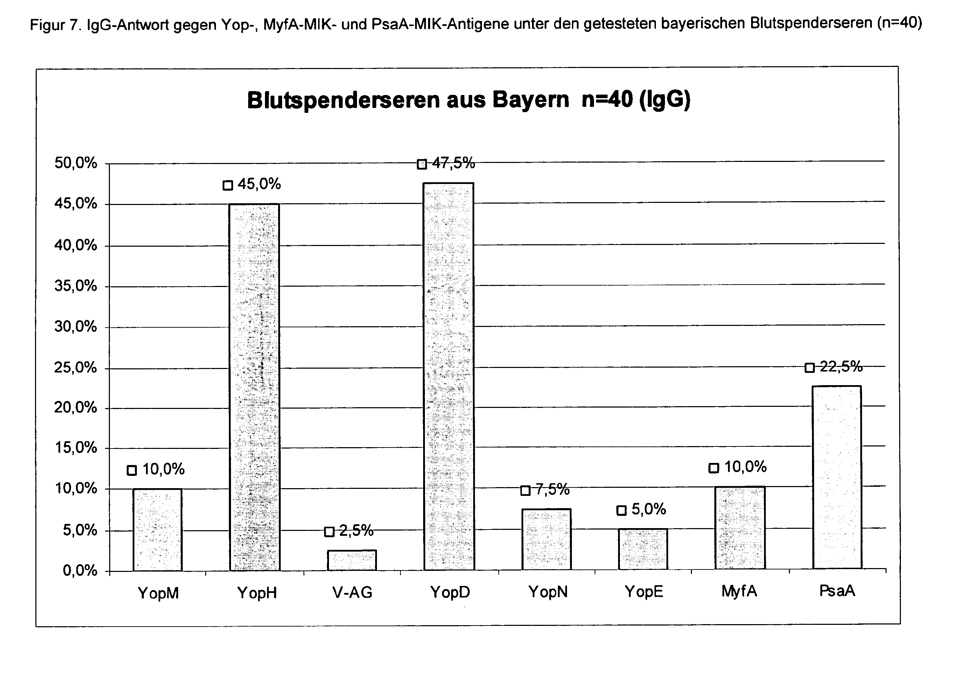

48-% (n=19) der getesteten Blutspenderseren aus Bayern (n=40) zeigten eine IgG-Reaktivität mit YopD (Fig. 7; Tabelle 1). Acht Serumproben wiesen eine IgG-Antwort auf, die nicht gegen YopD sondern ausschließlich gegen YopH (n=4), YopH und YopN (n=1), YopH und V-AG (n=1), YopM (n=1) und PsaA-MIK (n=1) gerichtet war. Drei der Proben reagierten mit den beiden neuen Yersinien-Antigene MyfA-MIK (n=4) und PsaA-MIK (n=9; Tab. 1).

In keinem der getesteten Seren konnten IgM-Antikörper gegen YopD nachgewiesen werden (Tab. 1). Drei Yop-IgG-positive Seren (n=19) wiesen einen isolierten IgM-Titer gerichtet gegen YopH (n=2; Serum Nr. 1087: hohe IgG-Titer gegen YopD und PsaA; Serum Nr. 1099: hohe IgG-Titer gegen YopD) oder PsaA (n=1; Serum Nr. 1083: hohe IgG-Titer gegen YopD, MyfA-MIK und PsaA-MIK) auf. Keine der getesteten Proben reagierte mit MyfA-MIK (Tab. 1).

Sieben YopD-IgG-positive Serumproben aus Blutspendern (n=19) zeigten einen IgA-Titer gegen YopD (Tab. 1). Drei der getesteten Seren reagierten mit PsaA-MIK. Interessanterweise zeigte die Yop-IgG-negative Serumprobe Nr. 1080 eine sehr schwache Reaktivität mit PsaA. Die YopD- und PsaA-MIK IgG-hoch positiven Serumproben Nr. 1096 und Nr. 1110 wiesen auch eine starke (Serum Nr. 1096) bzw. schwache (Serum Nr. 1110) IgA-Antwort gegen PsaA-MIK (Tab. 1) auf. Keiner der getesteten Proben reagierte mit MyfA-MIK (Tab. 1).

### Yersiniose-Patientenseren aus Finnland

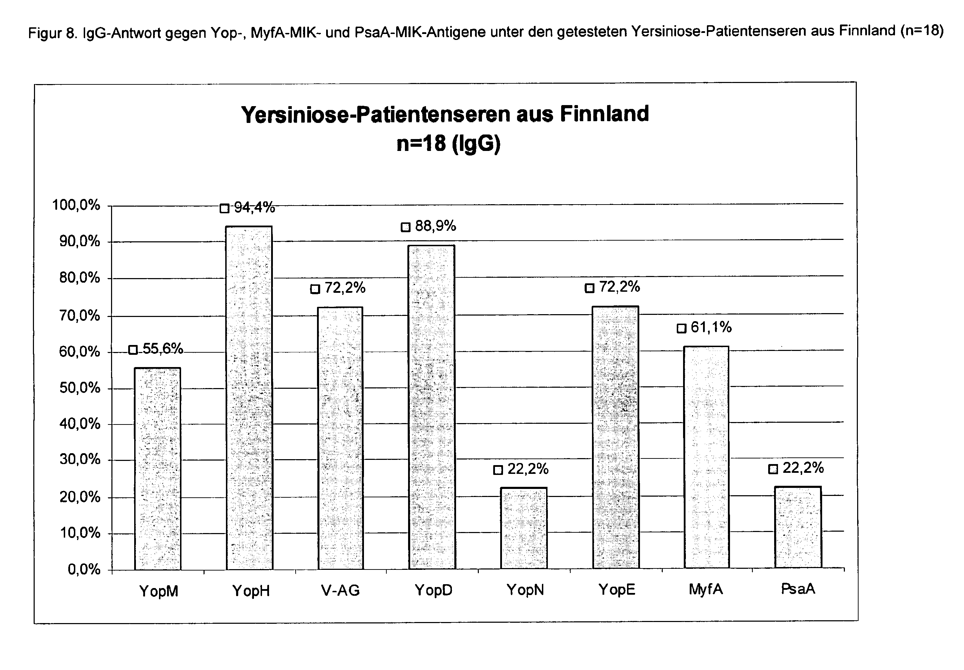

17 (90-%) der getesteten Patientenseren aus Finnland (n=18) zeigten eine IgG-Antwort gegen YopD (Fig. 8; Tab. 2). Elf der Proben reagierten mit MyfA-MIK und vier mit PsaA-MIK (Fig. 8; Tab. 2).

15 der getesteten Seren hatten IgM-Antikörper gegen YopD (Tab. 2). Ein Serum (Nr. 52) zeigte auch eine starke IgM-Antwort gegen PsaA-MIK (Tab. 2).

17 Serumproben aus Yersiniose-Patienten (n=18) wiesen einen IgA-Titer gegen YopD (Tab. 2) auf. Drei der getesteten Seren reagierten auch mit PsaA-MIK (Nr. 23, 24 und 40; Tab. 2).

### Yersiniose-Patientenseren aus Deutschland

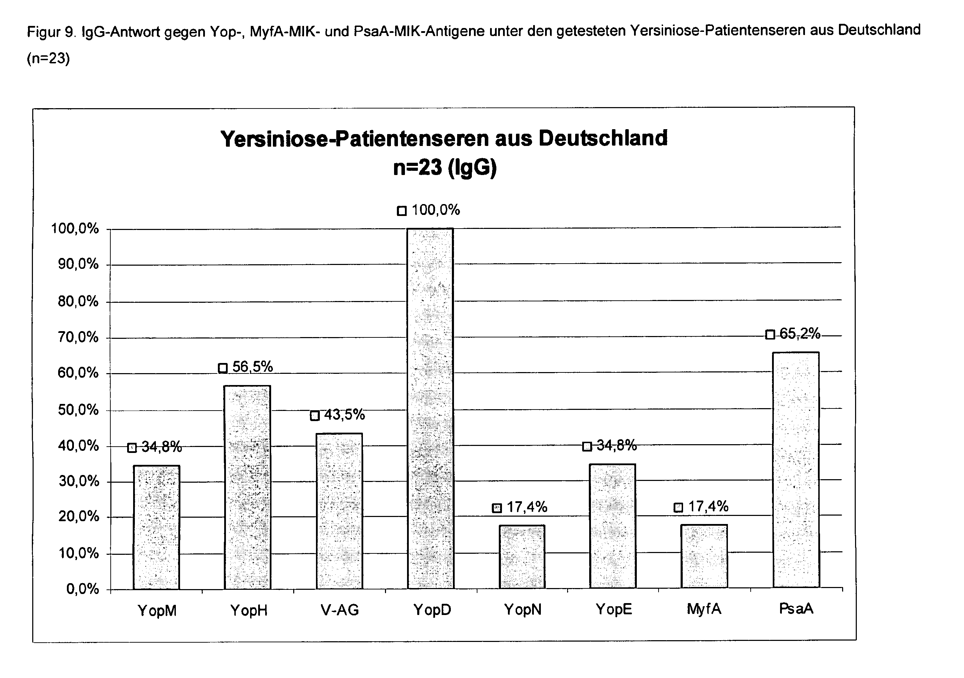

Alle (n=23) getesteten Yersiniose-Patientenseren aus Deutschland zeigten eine IgG-Reaktivität mit YopD (Fig. 9; Tab. 3). 15 Serumproben (65%) wiesen zusätzlich eine IgG-Antwort gegen PsaA-MIK auf. Vier der Proben reagierten mit dem Antigen MyfA-MIK (Fig. 9; Tab. 3). Interessanterweise reagierten zwei der Serumproben (Serum 1038 und 1025) mit den beiden Antigenen MyfA-MIK und PsaA-MIK. Jedoch war die IgG-Antwort gegen MyfA-MIK deutlich schwächer im Vergleich zu PsaA-MIK (Tab. 3).

Nur in zwei der getesteten Seren (n=23) wurde eine niedrige IgM-Antwort gegen YopD und in drei Seren gegen YopE nachgewiesen (Tab. 3). Ein Serum, Nr. 986, reagierte sehr stark mit PsaA-MIK (Tab. 3).

Alle getesteten Yersiniose-Patientenseren wiesen eine IgA-Antwort gegen YopD (Tab. 3) auf. Eine der Proben reagierte auch mit MyfA-MIK (Serum Nr. 976) und eine andere mit PsaA-MIK (Serum Nr. 1025). Diese Seren zeigten auch eine starke IgG-Antwort gegen MyfA-MIK bzw. PsaA-MIK (Tab. 3).

**Tabelle 1. Die IgG-, IgM- und IgA-Antwort gegen rekombinant hergestellte YopM-, YopH-, V-AG-, YopD, YopN-, YopE-, MyfA-MIK- und PsaA-MIK -Antigene unter den getesteten Blutspenderseren aus Bayern (n=40). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| **Serum** | **YopM** | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **1075** | 1 | 4 | | 4 | | 2 | 4 | 4 |
| **1076** | | 1 | | | 1 | | | |
| **1077** | | 2 | | 4 | | 4 | 2 | |
| **1078** | | | | | | | | |
| **1079** | | | | | | | | |
| **1080** | | | | | | | | |
| **1081** | | | | | | | | |
| **1082** | | 1 | | 3 | | | | |
| **1083** | | | | 4 | 1 | | 4 | 2 |
| **1084** | | | | | | | | |
| **1085** | | 3 | | 1 | | | | |
| **1086** | | 3 | | 3 | | | | |
| **1087** | | | | 4 | | | | 4 |
| **1088** | | | | | | | | |
| **1089** | | | | | | | | |
| **1090** | | 2 | | | | | | |
| **1091** | | 2 | | | | | | |
| **1092** | | | | | | | | |
| **1093** | 2 | 1 | | 4 | | | | |
| **1094** | | | | | | | | 2 |
| **1095** | | | | | | | | |
| **1096** | | | | 3 | | | 3 | 4 |
| **1097** | | 1 | | 3 | | | | |
| **1098** | | 1 | | 1 | | | | |
| **1099** | | | | 3 | | | | |
| **1100** | | | | | | | | |
| **1101** | 1 | 3 | | 2 | | | | |
| **1102** | | 3 | | 2 | | | | 3 |
| **1103** | | 3 | | | | | | |
| **1104** | | 1 | | | | | | |
| **1105** | | | | | | | | |
| **1106** | | | | | | | | |
| **1107** | 1 | | | | | | | |
| **1108** | | | | 1 | | | | 3 |
| **1109** | | 2 | | 2 | 2 | | | |
| **1110** | | | | 2 | | | | 3 |
| **1111** | | | | | | | | |
| **1112** | | 2 | | 4 | | | | 2 |
| **1113** | | 2 | 2 | | | | | |
| **1114** | | | | 4 | | | | |

| **IgM-Antwort** | | | | | | | | |
|---|---|---|---|---|---|---|---|---|
| **1075** | | | | | | | | |
| **1076** | | | | | | | | |
| **1077** | | | | | | | | |
| **1078** | | | | | | | | |
| **1079** | | | | | | | | |
| **1080** | | | | | | | | |
| **1081** | | | | | | | | |
| **1082** | | | | | | | | |
| **1083** | | | | | | | | 2 |
| **1084** | | | | | | | | |
| **1085** | | | | | | | | |
| **1086** | | | | | | | | |
| **1087** | | 1 | | | | | | |
| **1088** | | | | | | | | |
| **1089** | | | | | | | | |
| **1090** | | | | | | | | |
| **1091** | | | | | | | | |
| **1092** | | | | | | | | |
| **1093** | | | | | | | | |
| **1094** | | | | | | | | |
| **1095** | | | | | | | | |
| **1096** | | | | | | | | |
| **1097** | | | | | | | | |
| **1098** | | | | | | | | |
| **1099** | | 4 | | | | | | |
| **1100** | | | | | | | | |
| **1101** | | | | | | | | |
| **1102** | | | | | | | | |
| **1103** | | | | | | | | |
| **1104** | | | | | | | | |
| **1105** | | | | | | | | |
| **1106** | | | | | | | | |
| **1107** | | | | | | | | |
| **1108** | | | | | | | | |
| **1109** | | | | | | | | |
| **1110** | | | | | | | | |
| **1111** | | | | | | | | |
| **1112** | | | | | | | | |
| **1113** | | | | | | | | |
| **1114** | | | | | | | | |

| **IgA-Antwort** | | | | | | | | |
|---|---|---|---|---|---|---|---|---|
| **1075** | | | | | | | | |
| **1076** | | | | | | | | |
| **1077** | | | | 1 | | | | |
| **1078** | | | | | | | | |
| **1079** | | | | | | | | |
| **1080** | | | | | | | | 1 |
| **1081** | | | | | | | | |
| **1082** | | | | 1 | | | | |
| **1083** | | | | | | | | |
| **1084** | | | | | | | | |
| **1085** | | | | | | | | |
| **1086** | | | | | | | | |
| **1087** | | | | 4 | | | | |
| **1088** | | | | | | | | |
| **1089** | | | | | | | | |
| **1090** | | | | | | | | |
| **1091** | | | | | | | | |
| **1092** | | | | | | | | |
| **1093** | | | | 1 | | | | |
| **1094** | | | | | | | | |
| **1095** | | | | | | | | |
| **1096** | | | | | | | | 3 |
| **1097** | | | | 3 | | | | |
| **1098** | | | | | | | | |
| **1099** | | | | | | | | |
| **1100** | | | | | | | | |
| **1101** | | | | | | | | |
| **1102** | | | | | | | | |
| **1103** | | | | | | | | |
| **1104** | | | | | | | | |
| **1105** | | | | | | | | |
| **1106** | | | | | | | | |
| **1107** | | | | | | | | |
| **1108** | | | | | | | | |
| **1109** | | | | | | | | |
| **1110** | | | | 3 | 2 | | | 1 |
| **1111** | | | | | | | | |
| **1112** | | | | | | | | |
| **1113** | | | | | | | | |
| **1114** | | | | 1 | | | | |

**Tabelle 2. Die IgG-, IgM- und IgA-Antwort gegen rekombinant hergestellte YopM-, YopH-, V-AG-, YopD, YopN-, YopE-, MyfA-MIK- und PsaA-MIK -Antigene unter den getesteten Yersiniose-Patientenseren aus Finnland (n=18). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| **YopM** | | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **1** | 1 | 3 | 3 | 4 | 2 | 3 | 1 | |
| **5** | 2 | 4 | 1 | 4 | | 1 | 3 | |
| **6** | | 1 | 1 | 4 | | | | |
| **11** | | 3 | 1 | 3 | | 2 | 1 | |
| **16** | | 2 | 2 | 3 | | | | |
| **19** | | 3 | | 4 | | 2 | | |
| **23** | | 2 | | 3 | | | | 3 |
| **24** | 2 | 4 | 3 | 4 | | 3 | 1 | 4 |
| **34** | | 3 | | 3 | | 1 | 2 | |
| **35** | 2 | 3 | 3 | 4 | | 1 | | |
| **36** | 2 | 2 | 2 | 4 | | 1 | 1 | |
| **40** | 3 | 3 | 3 | 4 | 1 | 2 | 3 | 1 |
| **44** | | 3 | 1 | 3 | | 1 | | |
| **47** | | | | | | | | |
| **50** | 2 | 3 | | 4 | | 3 | 1 | 2 |
| **51** | 3 | 4 | 3 | 4 | 3 | 3 | 1 | |
| **52** | 1 | 4 | 2 | 4 | 2 | 3 | 1 | |
| **53** | 3 | 4 | 2 | 4 | | | 1 | |

| **IgM-Antwort** | | | | | | | | |
|---|---|---|---|---|---|---|---|---|
| **1** | | 1 | | 4 | | | | |
| **5** | | | | 3 | | | | |
| **6** | | | | 2 | | | | |
| **11** | | | | 1 | | | | |
| **16** | | | | 1 | | | | |
| **19** | | | | 3 | | | | |
| **23** | | | | | | | | |
| **24** | | | | 3 | | | | |
| **34** | | | | | | | | |
| **35** | | | | 4 | | | | |
| **36** | | | | 3 | | | | |
| **40** | | | | 1 | | | | |
| **44** | | | | 2 | | | | |
| **47** | | | | | | | | |
| **50** | | | | 1 | | | | |
| **51** | | | | 3 | | | | |
| **52** | | | | 2 | | | | 3 |
| **53** | | | | 4 | | | | |

| **IgA-Antwort** | | | | | | | | |
|---|---|---|---|---|---|---|---|---|
| **1** | | | | 2 | | | | |
| **5** | | | | 2 | | | | |
| **6** | | | | 4 | | | | |
| **11** | | | | 1 | | | | |
| **16** | | | | 2 | | | | |
| **19** | | | | 2 | | | | |
| **23** | | | | 2 | | | | 4 |
| **24** | | 1 | | 4 | | | | 3 |
| **34** | | | | 2 | | | | |
| **35** | | | | 3 | | | | |
| **36** | | | | 3 | | | | |
| **40** | 1 | 2 | 1 | 4 | 2 | 2 | | 1 |
| **44** | | 1 | | 4 | | | | |
| **47** | | | | | | | | |
| **50** | | | | 3 | | | | |
| **51** | | 1 | | 2 | | | | |
| **52** | | | | 2 | 1 | | | |
| **53** | | 3 | | 4 | | | | |

**Tabelle 3. Die IgG-, IgM- und IgA-Antwort gegen rekombinant hergestellte YopM-, YopH-, V-AG-, YopD, YopN-, YopE-, MyfA-MIK- und PsaA-MIK -Antigene unter den getesteten Yersiniose-Patientenseren aus Deutschland (n=23). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| **YopM** | | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **262** | | | | 2 | | | | 2 |
| **327** | | 4 | 2 | 4 | 3 | 2 | | 2 |
| **1534** | 1 | 3 | 3 | 4 | 1 | 4 | 3 | |
| **976** | | 4 | | 4 | | 4 | 2 | |
| **1038** | 2 | 3 | 2 | 3 | | | 1 | 2 |
| **158** | 2 | 3 | 1 | 4 | | 2 | | 3 |
| **58** | 2 | 3 | | 4 | | | | |
| **321** | | | | 4 | | | | 2 |
| **326** | | 4 | 2 | 4 | 2 | 2 | | 2 |
| **333** | | | 1 | 4 | | | | 2 |
| **1025** | | 3 | | 4 | | | 1 | 4 |
| **1547** | | | | 1 | | | | |
| **111** | | | | 4 | | | | 2 |
| **1** | 3 | 4 | 3 | 4 | | 4 | | 3 |
| **252** | 1 | 4 | 2 | 4 | 2 | 4 | | |
| **262** | | | | 3 | | | | 3 |
| **113** | | 4 | 2 | 4 | | 3 | | |
| **986** | | 4 | | 4 | | | | |
| **320** | 2 | | | 2 | | | | |
| **1748** | 1 | | 1 | 4 | | | | 3 |
| **1176** | | 3 | | 3 | | | | 3 |
| **1801** | | | | 4 | | | | 3 |
| **1810** | | | | 4 | | | | 3 |

| **IgM-Antwort** | | | | | | | | |
|---|---|---|---|---|---|---|---|---|
| | | | | | | | | |
| **262** | | | | | | | | |
| **327** | | | | | | | | |
| **1534** | | | | | | | | |
| **976** | | | | | | | | |
| **1038** | | | | | | 1 | | |
| **158** | | | | | | 1 | | |
| **58** | | | | | | 1 | | |
| **321** | | | | 1 | | | | |
| **326** | | | | | | | | |
| **333** | | | | 1 | | | | |
| **1025** | | | | | | | | |
| **1547** | | | | | | | | |
| **111** | | | | | | | | |
| **1** | | | | | | | | |
| **252** | | | | | | | | |
| **262** | | | | | | | | |
| **113** | | | | | | | | |
| **986** | | | | | | | | 4 |
| **320** | | | | | | | | |
| **1748** | | | | | | | | |
| **1176** | | | | | | | | |
| **1801** | | | | | | | | |
| **1810** | | | | | | | | |

| **IgA-Antwort** | | | | | | | | |
|---|---|---|---|---|---|---|---|---|
| **262** | | | | 2 | | | | |
| **327** | | | | 2 | | | | |
| **1534** | | | | 2 | | | | |
| **976** | | | | 2 | | | 1 | |
| **1038** | | | | 2 | | | | |
| **158** | | | | 3 | | | | |
| **58** | | | | 2 | | | | |
| **321** | | | | 3 | | | | |
| **326** | | | | 2 | | | | |
| **333** | | | | 3 | | | | |
| **1025** | | | | 2 | | | | 3 |
| **1547** | | | | 1 | | | | |
| **111** | | | | 3 | | | | |
| **1** | 3 | 3 | | 3 | | | | |
| **252** | | | | 2 | | | | |
| **262** | | | | 3 | | | | |
| **113** | | | | 3 | | | | |
| **986** | | | | 2 | | | | |
| **320** | 2 | | | 1 | | | | |
| **1748** | | | | 3 | | 2 | | |
| **1176** | | | | 4 | | | | |
| **1801** | | | | 4 | | | | |
| **1810** | | | | 4 | | | | |

## Patentansprüche

1. Vorrichtung zum serologischen Nachweis einer Infektion mit Yersinia, **dadurch gekennzeichnet, dass** diese Vorrichtung wenigstens ein Antigen, ausgewählt aus der Gruppe von Antigenen bestehend aus folgender Gruppe: YopD, YopH, YopM, YopE, V-AG und/oder YopN oder ein Fragment eines dieser Antigene mit mindestens acht aufeinanderfolgenden Aminosäuren und weiterhin eines von zwei Proteinen ausgewählt aus MyfA und PsaA oder Fragmente eines dieser beiden Proteine mit mindestens acht aufeinanderfolgenden Aminosäuren aufweist.

2. Vorrichtung nach Anspruch 1, **dadurch gekennzeichnet, dass** diese Vorrichtung sowohl das Protein MyfA, wie auch das Protein PsaA oder Fragmente dieser Proteine mit mindestens acht aufeinanderfolgenden Aminosäuren aufweist.

3. Vorrichtung nach Anspruch 1 oder 2, **dadurch gekennzeichnet, dass** diese Vorrichtung das Antigen YopD oder ein Fragment dieses Proteins mit mindestens acht aufeinanderfolgenden Aminosäuren aufweist.

4. Vorrichtung nach einem der Ansprüche 1-3, **dadurch gekennzeichnet, dass** die Fragmente der als Antigene eingesetzten Proteine wenigstens 12 aufeinander folgende Aminosäuren des jeweiligen Antigens aufweisen.

5. Vorrichtung nach einem der Ansprüche 1-3, **dadurch gekennzeichnet, dass** die Fragmente der als Antigene eingesetzten Proteine wenigstens 20 aufeinanderfolgende Aminosäuren des jeweiligen Antigens aufweisen.

6. Vorrichtung nach einem der Ansprüche 1-3, **dadurch gekennzeichnet, dass** die Fragmente der als Antigen eingesetzten Proteine wenigstens 30 Aminosäuren des jeweiligen Antigens aufweisen.

7. Vorrichtung nach einem der Ansprüche 1-6, **dadurch gekennzeichnet, dass** es sich um einen diagnostischen Kit handelt.

8. Vorrichtung nach Anspruch 7, **dadurch gekennzeichnet, dass** es sich um einen ELISA-Test handelt.

9. Vorrichtung nach Anspruch 7, **dadurch gekennzeichnet, dass** es sich um einen Line-Test handelt.

10. Vorrichtung nach Anspruch 7, **dadurch gekennzeichnet, dass** es sich um Immunoblots, Bead-Based-Assays oder Mikroarrays handelt.

11. Vorrichtung nach einen der Ansprüche 1-10, **dadurch gekennzeichnet, dass** sie dem Nachweis einer Infektion mit Humanpathogenen Yersinia-Species dient.

12. Vorrichtung nach einem der Ansprüche 1-10, **dadurch gekennzeichnet, dass** sie zum serologischen Nachweis einer Infektion verursacht durch *Yersinia enterocolitica* und/oder *Yersinia pseudotuberculosis* dient.

13. Vorrichtung nach einem der Ansprüche 1-10, **dadurch gekennzeichnet, dass** sie zur serologischen Unterscheidung einer Infektion mit *Yersinia enterocolitica* von einer Infektion mit *Yersinia pseudotuberculosis* dient.

14. Verwendung des antigenen Proteins MyfA und/oder PsaA oder eines Fragmentes eines dieser Proteine mit wenigstens 8 aufeinanderfolgenden Aminosäuren zum serologischen Nachweis einer durch *Yersinia enterocolitica* verursachten Infektion und/oder einer durch *Yersinia pseudotuberculosis* verursachten Infektion.
